# EUROPEAN PATENT SPECIFICATION

(11) **EP 3 310 302 B1**
(45) Date of publication and mention of the grant of the patent: **10.07.2024**
(21) Application number: 16815220.5
(22) Date of filing: 22.06.2016
(51) Int. Cl.: A61F 2/24

(54) **ACTIVELY CONTROLLABLE HEART VALVE IMPLANT**
AKTIV STEUERBARES HERZKLAPPENIMPLANTAT
IMPLANT DE VALVE CARDIAQUE POUVANT ÊTRE COMMANDÉ DE MANIÈRE ACTIVE

(30) Priority: 22.06.2015 US 201562182820 P; 23.06.2015 US 201562183451 P
(43) Date of publication of application: 25.04.2018
(73) Proprietor: Edwards Lifescience Cardiaq LLC, Irvine, CA 92614 (US)
(72) Inventor: GREENE, James, L., Minneapolis, MN 55402 (US); JIMENEZ, Jorge, Atlanta, GA 30327 (US); CARTLEDGE, Richard, Boca Raton, FL 33432 (US); DEVILLE, Derek, Dee, Coral Gables, FL 33156 (US); SMITH, Kevin, W., Coral Gables, FL 33156 (US)
(74) Representative: Eisenführ Speiser
(86) International application number: PCT/US2016/038776
(87) International publication number: WO 2016/209970

(56) References cited:
- WO-A1-2016/065158
- WO-A1-2017/096157
- US-A1- 2008 208 327
- US-A1- 2009 099 653
- US-A1- 2010 256 723
- US-A1- 2013 053 950
- US-A1- 2013 166 017
- US-A1- 2013 166 017
- US-A1- 2013 338 766
- US-A1- 2014 214 159
- US-A1- 2014 296 975
- US-A1- 2014 358 224
- US-B2- 7 993 394

## Description

### Technical Field

The present invention lies in the field of heart valve implants (including mitral, aortic, pulmonary, and tricuspid), and systems for controlling and implanting heart valves.

The human heart can suffer from various valvular diseases, which can result in significant malfunctioning of the heart and ultimately require replacement of the native heart valve with an artificial valve. There are a number of known artificial valves and a number of known methods of implanting these artificial valves in humans.

One method of implanting an artificial heart valve in a human patient is via open-chest surgery, during which the patient's heart is stopped and the patient is placed on cardiopulmonary bypass (using a so-called "heart-lung machine"). In one common surgical procedure, the diseased native valve leaflets are excised and a prosthetic valve is sutured to the surrounding tissue at the native valve annulus. Because of the trauma associated with the procedure and the attendant duration of extracorporeal blood circulation, some patients do not survive the surgical procedure or die shortly thereafter. It is well known that the risk to the patient increases with the amount of time required on extracorporeal circulation. Due to these risks, a substantial number of patients with defective native valves are deemed inoperable because their condition is too frail to withstand the procedure.

Because of the drawbacks associated with conventional open-chest surgery, percutaneous and minimally-invasive surgical approaches are in some cases preferred. In one such technique, a prosthetic valve is configured to be implanted in a much less invasive procedure by way of catheterization. For instance, U.S. Patent Nos. 7,393,360, 7,510,575, and 7,993,394 describe collapsible transcatheter prosthetic heart valves that can be percutaneously introduced in a compressed state on a catheter and expanded to a functional size at the desired position by balloon inflation or by utilization of a self-expanding frame or stent.

Various heart valve replacement devices exist in the art and, during the past decade, advancements in valve replacement implants have been achieved. Many of these advancements have occurred with those implants delivered percutaneously in a compressed state on a catheter and, with outer sheath retraction, self-expand to a given extent for implantation. Some implants are made of entirely self-expanding structures. Other implants partially self-expand and then are further expanded by force. Such dual-expansion implants can be made from a single, substantially cylindrical, lattice structure having a pre-defined (e.g., heat-set) initial shape that is smaller than the intended implantation diameter of an anatomic orifice, such as a vessel or heart valve. The lattice can be made of nitinol, for example. A lattice of non-self-expanding material can also be used, for example, of a cobalt chromium material. Within the lattice there can be a set of adjustable expansion devices that place respective forces upon the lattice to elastically and/or plastically deform the lattice to a size that is even greater than the pre-defined shape. One example of the expansion devices is a set of jack screws that are controlled by rotating drive wires (which wires extend from the implant location to the environment outside the patient and terminate, for example, at an electronic delivery control handle). As shown in U.S. Patent Application Publication Nos. 2013/0046373, 2013/0166017, and 2014/0296962, these rotating wires are initially connected to a respective jack screw and rotation of each wire causes a corresponding rotation of the jack screw. With the jack screws being connected to the lattice on each of their opposing ends (for example, through a threaded connection on one end and a freely rotating but longitudinally fixed connection on the other), rotation in one direction expands the circumference of the lattice and rotation in the other direction contracts the lattice. These control wires can be connected to the delivery handle with temporary securement structures that keep the wires rotationally connected to the respective jack screw until implantation and release of the replacement valve is desired. Before being disconnected, the control wires can reversibly expand and contract the lattice as the surgeon desires for optimal placement in the installation location. In other words, such implants can be repositioned before final deployment. When the implant is positioned in a final desired orientation, the drive wires are disconnected from all of the jack screws and are removed from the patient.

One advantage that such implants have over entirely self-expanding lattices is that these implants can be carefully expanded and also can provide feedback to the operator as to the device diameter and forces encountered from surrounding tissue. In contrast, entirely self-expanding implants continuously expand and apply an outwardly directed force where the lattice is implanted. The final diameter of the implant is not finely controllable or adjustable. Expansion of the tissue could lead to paravalvular leakage, movement of the implant, and/or embolism, all of which are undesirable.

Another feature of lattice implants that, upon deployment, first self-expand when removed from the installation catheter and then are forcibly expanded into the delivery site (referred to as self-expanding/forcibly expanding) is the fact that the force imparted against the tissue can be measured (and/or calculated) and either minimized or set to a desired value. While rotating the drive wires, any torque applied to the drive wires can be measured and determined with an implant delivery and deployment system having sensors (e.g., electronic sensors) that measure various parameters, such as current draw for example. Rotation of the drive wires for expanding the implant can be halted when a value of the determined torque is reached.

Delivery of implants in the art for replacement or repair of a heart valve can be achieved over different avenues. One percutaneous way that implant delivery can occur is through the aorta, where the entry site in the patient is located adjacent the femoral artery, referred to as the transfemoral (TF) approach. Another route to implantation of a replacement valve is through a transapical approach. Aortic replacement valves installed in these manners are referred to as Transcatheter Aortic Valve Replacement (TAVR) and Transcatheter Aortic Valve Implantation (TAVI) surgeries, which can be transapical. A third path through the septum of the heart is also possible and one such procedure is referred as a Transseptal (TS) Antegrade Transcatheter Aortic Valve Replacement.

For the treatment of mitral valve disease, Transcatheter Mitral Valve Replacement (TMVR) has been the subject of study, but has not been widely commercialized. Current TMVR techniques have several limitations. First, the size of the valves that are available for TMVR implant may not fit well. In particular, the mitral valve is not substantially circular, it has a D-shape with a long curving interface between the mitral valve's native leaflets. This is in contrast to the aortic valve, which is substantially circular. Also, the TMVR devices do not tend to allow for repositioning of the implant once it has been deployed in place. Next, the final expanded diameter of the known TMVR devices is pre-determined, making pre-sizing by a doctor a critical and difficult step. The physician must remotely assess the size of the diseased valve for selecting the correct implant. Migration of existing mitral valve implants is a significant clinical problem, potentially causing leakage and/or compromising necessary vascular supply. In such situations, emergency open surgery can be required, and/or it can lead to an unstable seal and/or migration.

No commercially approved transcatheter mitral valve exists. Some are being studied but there is no replacement mitral valve that can be fully repositioned during deployment and adjusted to better accommodate and seal a natural, diseased mitral valve. Thus, a need exists to overcome the problems with the prior art systems, designs, and processes as discussed above.

US 2014/0358224 A1 relates to a self-expanding wire frame for a pre-configured compressible transcatheter prosthetic cardiovascular valve, a combined inner valve-outer collar component system, and methods for deploying such a valve for treatment of a patient in need thereof.

### Disclosure

Embodiments of the systems, apparatuses, and methods described herein relate to an actively controllable implant or heart valve implant and methods of controlling same that overcome the hereinafore-mentioned disadvantages of the heretofore-known devices and methods of this general type and that provide such features with the ability to be fully repositioned before final deployment.

Described herein are various systems, apparatuses, and methods for implanting replacement heart valves, which implants can be used in any valve of the heart. In some exemplary embodiments herein, implants for a stent graft, a valve, a mitral valve and associated system, apparatuses, and methods are shown and described.

As compared to other heart valves, in a diseased mitral valve, the tissue is relatively soft. This means that prior art self-expanding mitral implant valves which are oversized relative to the native mitral valve continuously provide an outward expanding force to the native mitral valve tissue. This force further expands the diseased tissue throughout the life of the implant. Such a result is not desirable for many reasons, e.g., leakage, movement, and/or embolization. Provided herein in some exemplary embodiments are heart valve (e.g., mitral valve) replacement implants that do not continuously provide an outwardly directed force after implantation. These implants have a self-expansion aspect but that self-expansion occurs only for a certain extent -- before or up to the native annulus of tissue surrounding the mitral valve. After the self-expansion occurs, the adjustable stent lattice portion of the implant is then forcibly expanded into the native annulus only to an extent to seat the implant within the annulus with no leakage occurring around the implant. This means that, when a correct and sufficient implanted status occurs, there will be no additional outwardly directed force imparted on the native annulus by the implant to cause further outwards expansion of that tissue over the life of the implant. This advantage over the prior art permits greater longevity. Also provided in some of the exemplary embodiments are optional structures that ensure a fluid-tight seal against each of the two sides of the valve being replaced. One exemplary implant-securing structure is a self-expanding, implant skirt attached to the adjustable stent lattice, having a material that is fluid-tight or resistant (or after being installed becomes fluid-tight), and, when released from the delivery catheter, springing open to occlude the side of the valve on which it resides. It essentially is in the form of an umbrella that contacts the side of the implant site on its entire circumference. Another independent exemplary implant-securing structure is a set of self-expanding, wall-retaining petals. These petals can be compressed within delivery wires while the implant is installed in the delivery catheter, can continue to be held radially inwards by the delivery wires, while the implant is being maneuvered and installed in an implant site, and spring open radially away from the central longitudinal axis of the implant when the delivery wires are released from the implant upon final deployment. In this way, the implant is adjustable and repositionable repeatedly in both the expansion and contraction directions up until final deployment. With both implant-securing structures on opposing sides of the implant, the petals, the implant skirt, and the adjustable stent lattice form an annulus having a concave U-shape that can entirely capture and hold therein the native valve annulus in a fluid-tight and leak-tight manner.

A further advantage of some of the embodiments of herein-described mitral valve implants relates to the size of the valve portion when the implant is secured in the native annulus. The native annulus of mitral valves are substantially D-shaped. One characteristic of a diseased mitral valve is that the annulus stretches outwardly, leaving the leaflets of the mitral valve unable to coapt and, thereby, impairing the functionality of the valve. In what is referred to as Mitral Valve Prolapse, one or both of the valve flaps are enlarged and do not close in an even manner. With improper closure, blood could flow backwards into the left atrium, referred to as Mitral Valve Regurgitation. With a stretching of the mitral valve annulus, even if a prior art implant is able to be secured therein, the size of that implant's valve opening may be too large for the patient. Some of the embodiments of the mitral valve implant herein provide a valve opening sized for optimal flow irrespective of the size of the diseased mitral valve annulus. These embodiments provide a fixed-sized valve opening contained within a variable outer annular skirt, the combined structures of the variably sized outer skirt and the fixed-sized valve being referred to herein as a trampoline valve. These exemplary implants, therefore, provide an ideal amount of flow through the valve of the implant in spite of the enlarged native mitral valve annulus. This means that, regardless of the final D-shaped diameter of the implanted stent lattice, the trampoline valve will have its own fixed maximum circular diameter, which improves valve function and durability. This feature allows a standard-sized valve to cover a large patient population with mitral valves of various sizes. The skirt can optionally have a downstream flair that creates a back seal when high pressure of ventricle contraction is imparted.

With the foregoing and other objects in view, there is provided, a mitral valve implant comprising a force-expanding mitral valve lattice having an interior orifice and a self-expanding valve trampoline lattice attached at the interior orifice of the force-expanding mitral valve lattice. The force-expanding mitral valve lattice is self-expandable to a first configuration and is force expandable from the first configuration to a second configuration.

With the objects in view, there is also provided a mitral heart valve implant system comprising a valve delivery system, a self-expanding and forcibly expanding mitral valve frame, a self-expanding implant skirt, wall-retaining wires, and a self-expanding valve trampoline lattice. The valve delivery system comprises a controller, a guidewire lumen connected to the controller and having a distal nosecone, a hollow external sheath surrounding the guidewire lumen, having a proximal end connected to the controller, and configured to retract proximally from an extended, valve-installed position, a given number of implant drive wires each having a distal drive wire connector, and hollow connector lumens equal in number to the given number and each respectively threaded on one of the drive wires and having a distal hollow connector sleeve. The self-expanding and forcibly expanding mitral valve frame defines a central axis and comprises proximal and distal jack screw strut pairs equal in number to the given number and disposed parallel to the central axis, intermediate struts equal in number to the given number and disposed parallel to the central axis, each intermediate strut disposed between two adjacent ones of the jack screw strut pairs, arms respectively connecting adjacent ones of the jack screw strut pairs and the intermediate struts, and a plurality of jack screws. The jack screws are each rotatably connected to one jack screw strut pair, form, together with the jack screw strut pairs, the intermediate struts, and the arms, an adjustable stent lattice having a ventricle side and an atrial side, are configured to reversibly forcibly expand and contract the adjustable stent lattice between a compressed state and an enlarged state for implantation of the mitral valve frame into a native mitral valve, and each have a driving connector shaped to removably mate and connect to the distal drive wire connector of one of the drive wires and be held connected thereto when the hollow connector sleeve is disposed about the mated driving connector and distal drive wire connector such that rotation of the drive wires correspondingly rotates the jack screws to forcibly expand or contract the adjustable stent lattice. The self-expanding implant skirt is attached to the ventricle side of the adjustable stent lattice. The implant skirt is configured to compress and be stored inside the external sheath and, when released from the external sheath at a native mitral valve, to self-expand and sealably position on tissue at a ventricular side of the native mitral valve. The wall-retaining wires are attached to the atrium side of the adjustable stent lattice and are configured to compress and be stored inside the external sheath and, when released from the external sheath at a native mitral valve, to self-expand on tissue at an atrial side of the native mitral valve. The self-expanding valve trampoline lattice is disposed inside and is connected to the adjustable stent lattice and comprises an expandable outer trampoline portion having a circumferential exterior connected to the interior of the adjustable stent lattice and a circumferential interior and an inner circumferential valve portion connected to the circumferential interior and extending inwardly from the circumferential interior to define an interior cylindrical portion and having a circular valve with internal valve leaflets disposed at the interior cylindrical portion.

With the objects in view, there is also provided a mitral heart valve implant system comprises a mitral valve lattice having a pre-set D-shaped cross-sectional configuration, defining an internal orifice, and comprising a plurality of jack screws configured to forcibly expand and contract the mitral valve lattice reversibly between a compressed configuration and an enlarged configuration, an outwardly flaring, self-expanding implant skirt attached to an exterior of the mitral valve lattice, the implant skirt shaped to be positioned on a ventricular side of a native mitral valve to secure the mitral valve lattice in the annulus of the native mitral valve, radially outwardly biased wall-retaining wires attached to the mitral valve lattice and shaped to be positioned on an atrial side of the native mitral valve to secure the mitral valve lattice in the annulus of the native mitral valve, a self-expanding valve trampoline lattice containing interior valve leaflets, the valve trampoline lattice disposed within the internal orifice of the mitral valve lattice and having a D-shape portion attached to the mitral valve lattice and a substantially cylindrical interior portion, wherein the valve leaflets are attached to the substantially cylindrical interior portion, and a delivery system comprising a plurality of implant drive wires temporarily connectable to the jack screws such that, when connected, rotation of the drive wires in one direction forcibly expands the mitral valve lattice towards the enlarged configuration and rotation of the drive wires in a direction opposite the one direction forcibly contracts the mitral valve lattice towards the compressed configuration.

With the objects in view, there is also provided a method for implanting a mitral heart valve including the steps of contracting a self-expanding and forcibly-expanding mitral valve of a shape-memory material set to a given shape to a reduced implantation size with a delivery system having drive wires, the mitral valve having an adjustable assembly with adjustable elements operatively connected to the drive wires such that, when the adjustable elements are adjusted by the drive wires, a configuration change in at least a portion of the mitral valve occurs, inserting the contracted mitral valve into a native mitral valve annulus in which the mitral valve is to be implanted, rotating the drive wires with the delivery system to forcibly expand the mitral valve into the native annulus, while rotating the drive wires, determining with the delivery system a torque applied to the drive wires, and stopping rotation of the drive wires based upon a value of the determined torque.

In accordance with another feature, the force-expanding mitral valve lattice is self-expandable to a first configuration and is force expandable from the first configuration to a second configuration.

In accordance with a further feature, the first configuration is one of circular and D-shaped.

In accordance with an added feature, the second configuration corresponds in shape to the one of circular and D-shaped first configuration.

In accordance with an additional feature, the mitral valve lattice comprises a plurality of jack screws configured to adjust expansion and contraction of a configuration of the mitral valve lattice.

In accordance with yet another feature, the mitral valve lattice is made of a shape memory material set shape to a given shape.

In accordance with yet a further feature, the valve trampoline has a cylindrical central region comprising valve leaflets.

In accordance with yet an added feature, the valve trampoline comprises a D-shaped portion.

In accordance with yet an additional feature, the valve leaflets have an inflow side and the D-shaped portion is located on an inflow side of the valve leaflets.

In accordance with again another feature, the mitral valve lattice has an exterior and there is provided an outwardly flaring implant skirt attached to the exterior of the mitral valve lattice and shaped to be positioned on a side of a native mitral valve.

In accordance with again a further feature, there are provided wall-retaining wires attached to the mitral valve lattice and shaped to be positioned on a side of the native mitral valve.

In accordance with again an added feature, the wall-retaining wires are in the shape of petals and have a pre-set, radially outward, memory shape to impart a force on the side of the native mitral valve when the mitral valve lattice is expanded within an annulus of the native mitral valve.

In accordance with again an additional feature, the implant skirt is a left ventricle implant skirt shaped to be positioned on a ventricular side of the native mitral valve when the mitral valve lattice is expanded within the annulus of the native mitral valve and the wall-retaining wires are left-atrium wall-retaining wires shaped to be positioned on an atrial side of the native mitral valve when the mitral valve lattice is expanded within the annulus of the native mitral valve.

In accordance with still another feature, the implant skirt is a left atrium implant skirt shaped to be positioned on an atrial side of the native mitral valve when the mitral valve lattice is expanded within the annulus of the native mitral valve and the wall-retaining wires are left-ventricle wall-retaining wires shaped to be positioned on a ventricular side of the native mitral valve when the mitral valve lattice is expanded within the annulus of the native mitral valve.

In accordance with still a further feature, the mitral valve lattice has an inlet end and an outlet end and the valve trampoline is attached to the inlet end of the interior orifice.

In accordance with still an added feature, the mitral valve lattice has an inlet end and an outlet end and the valve trampoline is attached to the outlet end of the interior orifice.

In accordance with a concomitant feature, the adjustable stent lattice has a pre-set D-shaped cross-section and the exterior of the expandable outer trampoline portion is pre-set to a circumferential D-shape.

Although the systems, apparatuses, and methods are illustrated and described herein as embodied in an actively controllable heart valve implant and methods of controlling same, it is, nevertheless, not intended to be limited to the details shown because various modifications and structural changes may be made within the scope and range of equivalents of the claims. Additionally, well-known elements of exemplary embodiments will not be described in detail or will be omitted so as not to obscure the relevant details of the systems, apparatuses, and methods.

Additional advantages and other features characteristic of the systems, apparatuses, and methods will be set forth in the detailed description that follows and may be apparent from the detailed description or may be learned by practice of exemplary embodiments. Still other advantages of the systems, and apparatuses may be realized by any of the instrumentalities, or combinations particularly pointed out in the claims.

Other features that are considered as characteristic for the systems and apparatuses are set forth in the appended claims. As required, detailed embodiments of the systems and apparatuses are disclosed herein; however, it is to be understood that the disclosed embodiments are merely exemplary of the systems, apparatuses, and methods, which can be embodied in various forms. Therefore, specific structural and functional details disclosed herein are not to be interpreted as limiting, but merely as a basis for the claims and as a representative basis for teaching one of ordinary skill in the art to variously employ the systems, apparatuses, and methods in virtually any appropriately detailed structure. Further, the terms and phrases used herein are not intended to be limiting; but rather, to provide an understandable description of the systems, apparatuses, and methods. While the specification concludes with claims defining the systems and apparatuses of the invention that are regarded as novel, it is believed that the systems and apparatuses will be better understood from a consideration of the following description in conjunction with the drawing figures, in which like reference numerals are carried forward. The invention is defined by the appended claims.

### Brief Description of Drawings

The accompanying figures, where like reference numerals refer to identical or functionally similar elements throughout the separate views, which are not true to scale, and which, together with the detailed description below, are incorporated in and form part of the specification, serve to illustrate further various embodiments and to explain various principles and advantages all in accordance with the systems, apparatuses, and methods. Advantages of embodiments of the systems, apparatuses, and methods will be apparent from the following detailed description of the exemplary embodiments thereof, which description should be considered in conjunction with the accompanying drawings in which:
FIG. 1 is a fragmentary, side perspective view of an exemplary embodiment of a distal end of a delivery and deployment system for an actively controllable heart valve implant;

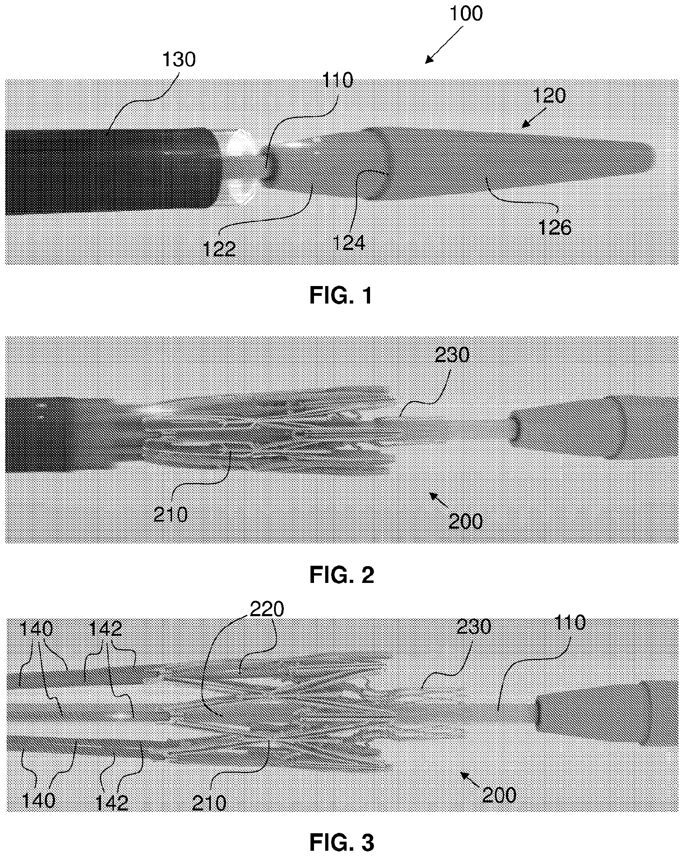

FIG. 2 is a fragmentary, side perspective view of the distal end of the delivery system of FIG. 1 with an exemplary embodiment of an actively controllable mitral valve replacement implant, with an implant skirt and a valve trampoline removed for clarity, in a pre-installation orientation with an outer catheter covering implant drive wires, with the drive wires connected to jack screws of a self-expanding and forcibly-expanding valve lattice, with a self-expanding valve trampoline lattice containing non-illustrated valve leaflets within an orifice of the valve lattice and attached to the valve lattice;
FIG. 3 is a fragmentary, side perspective view of the distal end of the delivery system of FIG. 1 with the mitral valve replacement implant of FIG. 2 in a pre-installation orientation with the outer sheath retracted from the implant drive wires;
FIG. 4 is an enlarged, fragmentary, side perspective view of the distal end of the delivery system of FIG. 1 with the mitral valve replacement implant of FIG. 3 in a pre-installation orientation with the mitral valve implant in a self-expanded, enlarged state, with the implant skirt and the valve trampoline transparent;
FIG. 5 is a fragmentary, perspective view of the distal end of the delivery system of FIG. 1 with the valve implant of FIG. 4 in a pre-installation orientation with the valve implant in a forcibly expanded, enlarged state;
FIG. 6 is a fragmentary, perspective view of the distal end of the delivery system of FIG. 1 with the mitral valve replacement implant of FIG. 5 in a fully-expanded, delivery orientation with the drive wires still engaged to the implant and constraining atrium wall retainers;
FIG. 7 is a fragmentary, perspective view of the distal end of the delivery system of FIG. 1 with the mitral valve replacement implant of FIG. 5 having the drive wires disengaged from the implant and from the atrium wall-retaining structures;
FIG. 8 is a fragmentary, perspective view of the distal end of the delivery system of FIG. 1 with the mitral valve replacement implant of FIG. 7 having the drive wires in a further retracted position from the implant and with the nosecone in a retracted state within the implant;
FIG. 9 is a ventricle-side elevational view of the mitral valve replacement implant of FIG. 8 with the mitral valve leaflets closed and with the D-shape of the self-expanding valve trampoline lattice visible;
FIG. 10 is an atrium-side elevational view of the mitral valve replacement implant of FIG. 8 with the valve leaflets in an almost-closed state;
FIG. 11 is a ventricle-side perspective view of the mitral valve replacement implant of FIG. 9 with the valve leaflets partially open;
FIG. 12 is an atrium-side perspective view of the mitral valve replacement implant of FIG. 9 with the valve in a substantially open state;
FIG. 13 is a side elevational view of the mitral valve replacement implant of FIG. 15 with attachment points connecting the skirt lattice and the skirt fabric to the adjustable stent lattice;
FIG. 14 is a fragmentary, perspective view of the delivery system of FIG. 1 for the actively controllable mitral valve replacement implant in a vertical cross-section of a human heart and with the guidewire in the left ventricle and the nosecone entering the left atrium of a ventricle-contracted heart;
FIG. 15 is a fragmentary, perspective view of the delivery system of FIG. 14 with the nosecone in the left ventricle of a ventricle-relaxed heart;
FIG. 16 is a fragmentary, perspective view of the delivery system of FIG. 15 with the outer sheath withdrawn into the left atrium and the mitral valve replacement implant beginning to show within a ventricle-contracted heart;
FIG. 17 is a fragmentary, perspective view of the delivery system of FIG. 16 with the outer sheath substantially withdrawn over the mitral valve replacement implant and the implant skirt in a first self-expanded orientation within the mitral valve orifice of a ventricle-relaxed heart;
FIG. 18 is a fragmentary, perspective view of the delivery system of FIG. 17 with the implant skirt in a second self-expanded orientation within the mitral valve orifice and with the drive wires of the mitral valve replacement implant partially visible within a ventricle-contracted heart;
FIG. 19 is a fragmentary, perspective view of the delivery system of FIG. 18 with the implant skirt in a third self-expanded orientation within the mitral valve orifice and with the drive wires of the mitral valve replacement implant visible within a ventricle-contracted heart;
FIG. 20 is a fragmentary, perspective view of the delivery system of FIG. 19 with the adjustable stent lattice in a forcibly expanded orientation within the mitral valve orifice of a ventricle-relaxed heart;
FIG. 21 is a fragmentary, perspective view of the delivery system of FIG. 20 with the adjustable stent lattice and the implant skirt in a fully expanded and implanted orientation within the mitral valve orifice of a ventricle-contracted heart before disconnection of the drive wires;
FIG. 22 is a fragmentary, perspective view of an exemplary embodiment of a sheath-constrained mitral valve replacement implant within a mitral valve orifice viewed from the left atrium with the mitral valve partially open, with the drive wires fully constraining the atrium retaining petals, and with the outer delivery sheath removed;
FIG. 23 is a fragmentary, perspective view of the sheath-constrained mitral valve replacement implant of FIG. 22 with the mitral valve fully open;
FIG. 24 is a fragmentary, perspective view of the mitral valve replacement implant of FIG. 23 with the sheath retracted and with the skirt of the mitral valve replacement implant in a self-expanded state with the mitral valve fully open and with the drive wires constraining the atrium retaining petals;
FIG. 25 is a fragmentary, perspective view of the mitral valve replacement implant of FIG. 24 with the mitral valve closed upon the skirt of the valve implant;
FIG. 26 is a fragmentary, perspective view of the mitral valve replacement implant of FIG. 25 in a fully self-expanded state with the mitral valve closed upon the mitral valve replacement implant;
FIG. 27 is a fragmentary, perspective view of the mitral valve replacement implant of FIG. 26 in a forcibly expanded state with the mitral valve partially open around the mitral valve replacement implant and with the replacement valve of the mitral valve replacement implant partially open;
FIG. 28 is a fragmentary, perspective view of the mitral valve replacement implant of FIG. 27 in still a further forcibly expanded state with the mitral valve partially closed around the mitral valve replacement implant and with the replacement valve of the mitral valve replacement implant almost fully open;
FIG. 29 is a fragmentary, perspective view of the mitral valve replacement implant of FIG. 28 in yet a further forcibly expanded state with the mitral valve open around the mitral valve replacement implant and with the replacement valve of the mitral valve replacement implant fully open;
FIG. 30 is a fragmentary, perspective view of the mitral valve replacement implant of FIG. 29 in another forcibly expanded state with the mitral valve open around the mitral valve replacement implant and with the replacement valve of the mitral valve replacement implant partially open;
FIG. 31 is a fragmentary, perspective view of the mitral valve replacement implant of FIG. 30 with the mitral valve closed around the mitral valve replacement implant and with the replacement valve of the mitral valve replacement implant closed around the delivery guidewire lumen;
FIG. 32 is a fragmentary, perspective view of the mitral valve replacement implant of FIG. 31 held open by the mitral valve replacement implant in a valve-implanted state and with the replacement valve of the mitral valve replacement implant in an almost fully open state;
FIG. 33 is a fragmentary, perspective view of the mitral valve replacement implant of FIG. 32 with the mitral valve replacement implant in the valve-implanted state and with the drive wire assembly retracted and disengaged to no longer constrain the atrium retaining petals;
FIG. 34 is a fragmentary, perspective view of the mitral valve replacement implant of FIG. 33 with the mitral valve replacement implant in the valve-implanted state, with the drive wire assembly disengaged and further retracted, and with the atrium retaining petals retained against the wall of the left atrium;
FIG. 35 is a fragmentary, perspective view of the mitral valve replacement implant of FIG. 34 with the mitral valve replacement implant in the valve-implanted state, with the replacement valve of the mitral valve replacement implant in an open state, and with the drive wire assembly completely retracted;
FIG. 36 is a fragmentary, perspective view of the mitral valve replacement implant of FIGS. 9 to 13 in a skirt-and-valve-expanded state approximately equivalent to the view of the mitral valve replacement implant of FIG. 26 and with the replacement valve in a substantially open state;
FIG. 37 is a fragmentary, perspective view of the mitral valve replacement implant of FIG. 36 in a skirt-and-valve-expanded state approximately equivalent to the view of the mitral valve replacement implant of FIG. 27 and with the replacement valve in an almost closed state;
FIG. 38 is a fragmentary, perspective view of the mitral valve replacement implant of FIG. 37 in a skirt-and-valve-expanded state approximately equivalent to the view of the mitral valve replacement implant of FIG. 29 and with the replacement valve in a partially open state;
FIG. 39 is a fragmentary, perspective view of the mitral valve replacement implant of FIG. 38 in a skirt-and-valve-expanded state between the views of the mitral valve replacement implant of FIGS. 31 and 32 and with the replacement valve in a substantially closed state;
FIG. 40 is a fragmentary, perspective view of the mitral valve replacement implant of FIG. 39 in a valve-implanted state approximately equivalent to the view of the mitral valve replacement implant of FIG. 33 with the replacement valve in a substantially open state and with the drive wires disengaged from the mitral valve replacement implant;
FIG. 41 is a fragmentary, perspective view of the mitral valve replacement implant of FIG. 40 with the drive wires removed and with the nosecone partially retracted;
FIG. 42 is a fragmentary, perspective view of the mitral valve replacement implant of FIG. 41 with the nosecone retracted partially through and closed upon by the leaflets of the mitral valve replacement implant;
FIG. 43 is a fragmentary, perspective view of the mitral valve replacement implant of FIG. 42 with the nosecone withdrawn from the mitral valve replacement implant and with the replacement valve in a substantially open state;
FIG. 44 is a fragmentary, perspective view of an embodiment of an actively controllable delivery or deployment system for an adjustable stent graft;
FIG. 45 is a fragmentary, perspective view of another exemplary embodiment of an actively controllable stent graft connected to the delivery system of FIG. 44 in a partially expanded state;
FIG. 46 is a fragmentary, perspective view of the stent graft of FIG. 45 in a fully expanded and ready-to-implant state;
FIG. 47 is a fragmentary, perspective view of the stent graft of FIG. 46 implanted at a target area with tissue-engagement hooks deployed;
FIG. 48 is a fragmentary, vertically cross-sectional and perspective view of the delivery system of FIG. 44 inserted through the apex of the heart and through the mitral valve annulus into the left atrium;
FIG. 49 is a fragmentary, vertically cross-sectional and perspective view of the heart of FIG. 48 with the delivery system deploying the stent graft of FIG. 45 as a replacement mitral valve implant with the implant partially expanded in the mitral valve annulus;
FIG. 50 is a fragmentary, horizontally cross-sectional and perspective view of the heart of FIG. 49;
FIG. 51 is a fragmentary, horizontally cross-sectional and perspective view of the heart of FIG. 49 with the delivery system having deployed hooks of the implant into the mitral valve annulus;
FIG. 52 is a fragmentary, horizontally cross-sectional and perspective view of the heart of FIG. 51 with the delivery system removed from the heart and the implant secured in the mitral valve annulus;
FIG. 53 is a fragmentary, perspective view of the actively controllable delivery system of FIG. 44;
FIG. 54 is a fragmentary, perspective view of another exemplary embodiment of an actively controllable heart valve replacement implant connected to the delivery system of FIG. 53 in a partially expanded state with the inner valve assembly removed;
FIG. 55 is a fragmentary, perspective view of the heart valve replacement implant of FIG. 54 in a fully expanded and ready-to-implant state with interior portions of the inner valve assembly removed;
FIG. 56 is a fragmentary, perspective view of the heart valve replacement implant of FIG. 55 implanted at a target area with tissue-engagement hooks and a skirt deployed and with interior portions of the inner valve assembly removed;
FIG. 57 is a fragmentary, top plan view of the heart valve replacement implant of FIG. 56 implanted at a target area with tissue-engagement hooks and a skirt deployed and with interior portions of the inner valve assembly;
FIG. 58 is a fragmentary, vertically cross-sectional and perspective view of the delivery system of FIG. 44 inserted through the apex of the heart and through the mitral valve annulus into the left atrium;
FIG. 59 is a fragmentary, vertically cross-sectional and perspective view of the heart of FIG. 58 with the delivery system deploying the replacement mitral valve implant of FIG. 57 with the implant partially expanded in the mitral valve annulus;
FIG. 60 is a fragmentary, horizontally cross-sectional and perspective view of the heart of FIG. 59;
FIG. 61 is a fragmentary, horizontally cross-sectional and perspective view of the heart of FIG. 60 with the delivery system having deployed hooks of the implant into the mitral valve annulus;
FIG. 62 is a fragmentary, horizontally cross-sectional and perspective view of the heart of FIG. 61 with the delivery system removed from the heart and the implant secured in the mitral valve annulus;
FIG. 63 is a fragmentary, perspective view of the actively controllable delivery system of FIG. 44;
FIG. 64 is a fragmentary, perspective view of another exemplary embodiment of an actively controllable heart valve replacement implant connected to the delivery system of FIG. 63 in a partially expanded state with the inner valve assembly removed;
FIG. 65 is a fragmentary, perspective view of the heart valve replacement implant of FIG. 64 in a fully expanded and ready-to-implant state with interior portions of the inner valve assembly removed;
FIG. 66 is a fragmentary, perspective view of the heart valve replacement implant of FIG. 65 implanted at a target area with tissue-engagement hooks and an upstream skirt deployed and with interior portions of the inner valve assembly removed;
FIG. 67 is a fragmentary, top plan view of the heart valve replacement implant of FIG. 66 implanted at a target area with tissue-engagement hooks, an upstream skirt, and a trampoline valve deployed and with valve leaflets removed;
FIG. 68 is a fragmentary, vertically cross-sectional and perspective view of the delivery system of FIG. 44 inserted through the apex of the heart and through the mitral valve annulus into the left atrium;
FIG. 69 is a fragmentary, vertically cross-sectional and perspective view of the heart of FIG. 68 with the delivery system deploying the replacement mitral valve implant of FIG. 67 with the implant partially expanded in the mitral valve annulus;
FIG. 70 is a fragmentary, horizontally cross-sectional and perspective view of the heart of FIG. 69 with the leaflets removed from the implant;
FIG. 71 is a fragmentary, horizontally cross-sectional and perspective view of the heart of FIG. 70 with the delivery system having deployed hooks of the implant into the mitral valve annulus;
FIG. 72 is a fragmentary, horizontally cross-sectional and perspective view of the heart of FIG. 71 with the delivery system removed from the heart and the implant secured in the mitral valve annulus;
FIG. 73 is an atrium-side elevational view of the mitral valve replacement implant of FIG. 64 to 67 and 69 to 72 with the valve leaflets in an almost-closed state and with hook fasteners extended radially outward;
FIG. 74 is a top plan view of an implant skirt frame and an adjustable stent lattice of the mitral valve replacement implant of FIG. 73;
FIG. 75 is a perspective view from a side of the skirt frame and adjustable stent lattice of FIG. 74;
FIG. 76 is an atrium-side perspective view of another exemplary embodiment of an actively controllable, mitral valve replacement implant with an implant skirt, wall-retaining petals, and an internal valve trampoline;
FIG. 77 is an atrium-side perspective view of an implant skirt frame, wall-retaining petals, a valve trampoline lattice, and an adjustable stent lattice of the mitral valve replacement implant of FIG. 76;
FIG. 78 is a substantially side elevational view of the mitral valve replacement implant of FIG. 76;
FIG. 79 is a substantially side elevational view of the implant skirt frame, the wall-retaining petals, the valve trampoline lattice, and the adjustable stent lattice of the mitral valve replacement implant of FIG. 76;
FIG. 80 is a ventricle-side perspective view of the mitral valve replacement implant of FIG. 76;
FIG. 81 is a ventricle-side perspective view of the implant skirt frame, the wall-retaining petals, the valve trampoline lattice, and the adjustable stent lattice of the mitral valve replacement implant of FIG. 76;
FIG. 82 is an installation-side perspective view of another exemplary embodiment of an actively controllable, trampoline-side installed, circular valve replacement implant with an implant skirt, covered opposing-side wall-retaining petals, and an internal valve trampoline;
FIG. 83 is an installation-side perspective view of an implant skirt frame, wall-retaining petals, a valve trampoline lattice, and an adjustable stent lattice of the circular valve replacement implant of FIG. 82;
FIG. 84 is a substantially side elevational view of the circular valve replacement implant of FIG. 82;
FIG. 85 is a substantially side elevational view of the implant skirt frame, the wall-retaining petals, the valve trampoline lattice, and the adjustable stent lattice of the circular valve replacement implant of FIG. 82;
FIG. 86 is a nosecone-side perspective view of the circular valve replacement implant of FIG. 82;
FIG. 87 is a nosecone-side perspective view of the implant skirt frame, the wall-retaining petals, the valve trampoline lattice, and the adjustable stent lattice of the circular valve replacement implant of FIG. 82;
FIG. 88 is an installation-side perspective view of another exemplary embodiment of an actively controllable, petal-side installed, circular valve replacement implant with an implant skirt, opposing-side wall-retaining petals, and an internal valve trampoline facing the implant skirt;
FIG. 89 is an installation-side perspective view of an implant skirt frame, wall-retaining petals, a valve trampoline lattice, and an adjustable stent lattice of the circular valve replacement implant of FIG. 88;
FIG. 90 is a substantially side elevational view of the circular valve replacement implant of FIG. 88;
FIG. 91 is a substantially side elevational view of the implant skirt frame, the wall-retaining petals, the valve trampoline lattice, and the adjustable stent lattice of the circular valve replacement implant of FIG. 88;
FIG. 92 is a nosecone-side perspective view of the circular valve replacement implant of FIG. 88;
FIG. 93 is a nosecone-side perspective view of the implant skirt frame, the wall-retaining petals, the valve trampoline lattice, and the adjustable stent lattice of the circular valve replacement implant of FIG. 88;
FIG. 94 is a nosecone-side perspective view of another exemplary embodiment of an actively controllable, circular valve replacement implant;
FIG. 95 is an installation-side perspective view of the circular valve replacement implant of FIG. 94;
FIG. 96 is a nosecone-side perspective view of another exemplary embodiment of an actively controllable, circular valve replacement implant with a trampoline valve;
FIG. 97 is an installation-side perspective view of the circular valve replacement implant of FIG. 96;
FIG. 98 is an installation-side perspective view of an adjustable stent lattice and valve trampoline lattice of the circular valve replacement implant of FIG. 96;
FIG. 99 is a nosecone-side perspective view of the adjustable stent lattice the and valve trampoline lattice of FIG. 98;
FIG. 100 is a nosecone-side perspective view of another exemplary embodiment of an actively controllable, circular valve replacement implant with an installation-side implant skirt;
FIG. 101 is an installation-side perspective view of the circular valve replacement implant of FIG. 100;
FIG. 102 is a side perspective view of an adjustable stent lattice and an implant skirt frame of the circular valve replacement implant of FIG. 100;
FIG. 103 is a nosecone-side perspective view of another exemplary embodiment of an actively controllable, circular valve replacement implant with an installation-side implant skirt and a trampoline valve; and
FIG. 104 is an installation-side perspective view of the circular valve replacement implant of FIG. 103.

### Best Mode for Carrying Out the Invention

As required, detailed embodiments of the systems, apparatuses, and methods are disclosed herein; however, it is to be understood that the disclosed embodiments are merely exemplary of the systems, apparatuses, and methods, which can be embodied in various forms. Therefore, specific structural and functional details disclosed herein are optional and not to be interpreted as limiting, but merely as a basis for the claims and as a representative basis for teaching one skilled in the art to variously employ the systems, apparatuses, and methods in virtually any appropriately detailed structure. Further, the terms and phrases used herein are not intended to be limiting; but rather, to provide an understandable description of the systems, apparatuses, and methods. While the specification concludes with claims defining the features of the systems and apparatuses that are regarded as novel, it is believed that the systems, apparatuses, and methods will be better understood from a consideration of the following description in conjunction with the drawing figures, in which like reference numerals are carried forward.

In the following detailed description, reference is made to the accompanying drawings which form a part hereof, and in which are shown by way of illustration embodiments that may be practiced. It is to be understood that other embodiments may be utilized and structural or logical changes may be made without departing from the scope. Therefore, the following detailed description is not to be taken in a limiting sense, and the scope of embodiments is defined by the appended claims.

Additionally, well-known elements of exemplary embodiments of the systems, apparatuses, and methods will not be described in detail or will be omitted so as not to obscure the relevant details of the systems, apparatuses, and methods.

Before the systems, apparatuses, and methods are disclosed and described, it is to be understood that the terminology used herein is for the purpose of describing particular embodiments only and is not intended to be limiting. The terms "comprises," "comprising," or any other variation thereof are intended to cover a non-exclusive inclusion, such that a process, method, article, or apparatus that comprises a list of elements does not include only those elements but may include other elements not expressly listed or inherent to such process, method, article, or apparatus. An element proceeded by "comprises ... a" does not, without more constraints, preclude the existence of additional identical elements in the process, method, article, or apparatus that comprises the element. The terms "including" and/or "having," as used herein, are defined as comprising (i.e., open language). The terms "a" or "an", as used herein, are defined as one or more than one. The term "plurality," as used herein, is defined as two or more than two. The term "another," as used herein, is defined as at least a second or more. The description may use the terms "embodiment" or "embodiments," which may each refer to one or more of the same or different embodiments.

The terms "coupled" and "connected," along with their derivatives, may be used. It should be understood that these terms are not intended as synonyms for each other. Rather, in particular embodiments, "connected" may be used to indicate that two or more elements are in direct physical or electrical contact with each other. "Coupled" may mean that two or more elements are in direct physical or electrical contact (e.g., directly coupled). However, "coupled" may also mean that two or more elements are not in direct contact with each other, but yet still cooperate or interact with each other (e.g., indirectly coupled).

For the purposes of the description, a phrase in the form "A/B" or in the form "A and/or B" or in the form "at least one of A and B" means (A), (B), or (A and B), where A and B are variables indicating a particular object or attribute. When used, this phrase is intended to and is hereby defined as a choice of A or B or both A and B, which is similar to the phrase "and/or". Where more than two variables are present in such a phrase, this phrase is hereby defined as including only one of the variables, any one of the variables, any combination of any of the variables, and all of the variables, for example, a phrase in the form "at least one of A, B, and C" means (A), (B), (C), (A and B), (A and C), (B and C), or (A, B and C).

Relational terms such as first and second, top and bottom, and the like may be used solely to distinguish one entity or action from another entity or action without necessarily requiring or implying any actual such relationship or order between such entities or actions. The description may use perspective-based descriptions such as up/down, back/front, top/bottom, and proximal/distal. Such descriptions are merely used to facilitate the discussion and are not intended to restrict the application of disclosed embodiments. Various operations may be described as multiple discrete operations in turn, in a manner that may be helpful in understanding embodiments; however, the order of description should not be construed to imply that these operations are order dependent.

Herein the relational terms "proximal" and "distal" are used. Meanings for these terms are to be determined in the context in which they are used. In various embodiments, where proximal and distal are used with regard to the delivery system and the implant to be deployed, the term "proximal" is in the direction towards the delivery handle and the user and away from the implant and term "distal" is in the direction away from the delivery handle and the user and towards the implant.

As used herein, the term "about" or "approximately" applies to all numeric values, whether or not explicitly indicated. These terms generally refer to a range of numbers that one of skill in the art would consider equivalent to the recited values (i.e., having the same function or result). In many instances these terms may include numbers that are rounded to the nearest significant figure. As used herein, the terms "substantial" and "substantially" means, when comparing various parts to one another, that the parts being compared are equal to or are so close enough in dimension that one skill in the art would consider the same. Substantial and substantially, as used herein, are not limited to a single dimension and specifically include a range of values for those parts being compared. The range of values, both above and below (e.g., "+/-" or greater/lesser or larger/smaller), includes a variance that one skilled in the art would know to be a reasonable tolerance for the parts mentioned.

It will be appreciated that embodiments of the systems, apparatuses, and methods described herein may be comprised of one or more conventional processors and unique stored program instructions that control the one or more processors to implement, in conjunction with certain non-processor circuits and other elements, some, most, or all of the functions of the devices and methods described herein. The non-processor circuits may include, but are not limited to, signal drivers, clock circuits, power source circuits, and user input and output elements. Alternatively, some or all functions could be implemented by a state machine that has no stored program instructions, or in one or more application specific integrated circuits (ASICs) or field-programmable gate arrays (FPGA), in which each function or some combinations of certain of the functions are implemented as custom logic. Of course, a combination of these approaches could also be used. Thus, methods and means for these functions have been described herein.

The terms "program," "software," "software application," and the like as used herein, are defined as a sequence of instructions designed for execution on a computer system or programmable device. A "program," "software," "application," "computer program," or "software application" may include a subroutine, a function, a procedure, an object method, an object implementation, an executable application, an applet, a servlet, a source code, an object code, any computer language logic, a shared library/dynamic load library and/or other sequence of instructions designed for execution on a computer system.

Herein various embodiments of the systems, apparatuses, and methods are described. In many of the different embodiments, features are similar. Therefore, to avoid redundancy, repetitive description of these similar features may not be made in some circumstances. It shall be understood, however, that description of a first-appearing feature applies to the later described similar feature and each respective description, therefore, is to be incorporated therein without such repetition.

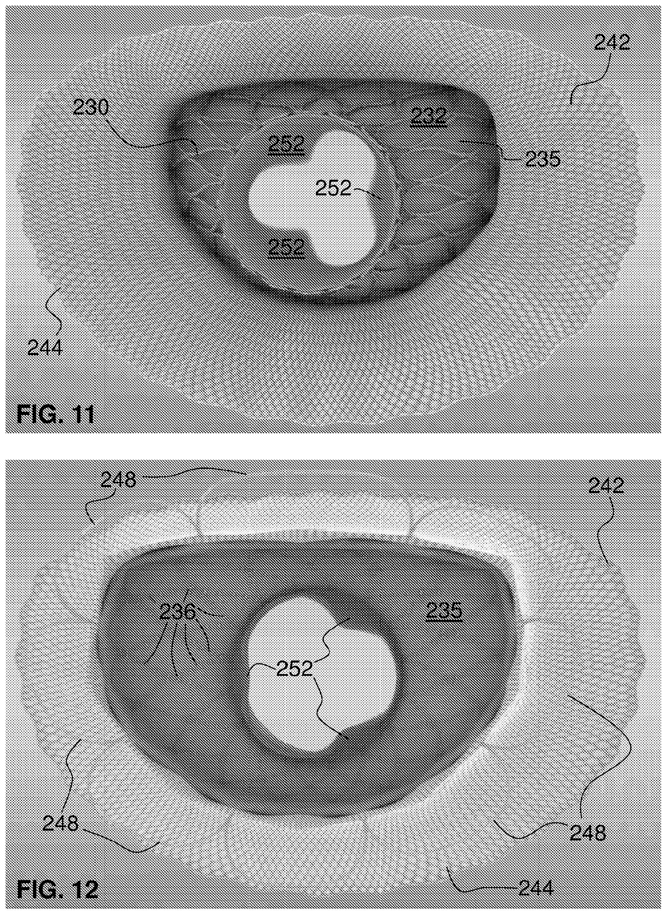

Described now are exemplary embodiments of the present invention. Referring now to the figures of the drawings in detail and first, particularly to FIGS. 1 to 13, there is shown a first exemplary embodiment of an actively controllable delivery and deployment system 100, for example, for an actively controllable mitral heart valve replacement implant 200. The delivery system 100 includes an inner elongate member that comprises a guidewire lumen 110, a nosecone 120 disposed at the distal end of the guidewire lumen 110, a hollow exterior sheath 130 surrounding the guidewire lumen 110 and shaped to smoothly connect to the proximal end of the nosecone 120, and a non-illustrated delivery and deployment handle connected to the guidewire lumen 110, to the exterior sheath 130, and to implant controls that are described in further detail below. The sheath-nosecone connection is established by forming the proximal end of the nosecone 120 with a taper 122 that ends with an abutting wall 124 at the proximal most end of the distal nosecone taper 126. The distal taper 126 has an outer diameter at the wall 124 that is substantially equal to the outer diameter of the distal end of the sheath 130 and the wall 124 has a height that is substantially equal to the thickness of the material of the hollow sheath 130.

The actively controllable mitral heart valve replacement implant 200 is, in FIG. 1, compressed within the sheath 130 and surrounding the guidewire lumen 110, as can be seen in FIG. 2, in which the sheath 130 has been retracted proximally to such an extent that the implant 200 can be seen in its entire longitudinal extent. Even though the implant 200 should be expanded further than the configuration shown in FIG. 2 when it is fully exposed from the sheath 130, the implant 200 is depicted in FIG. 2 in only a slightly expanded orientation as compared to the completely encased and compressed orientation of the non-visible implant 200 in FIG. 1.

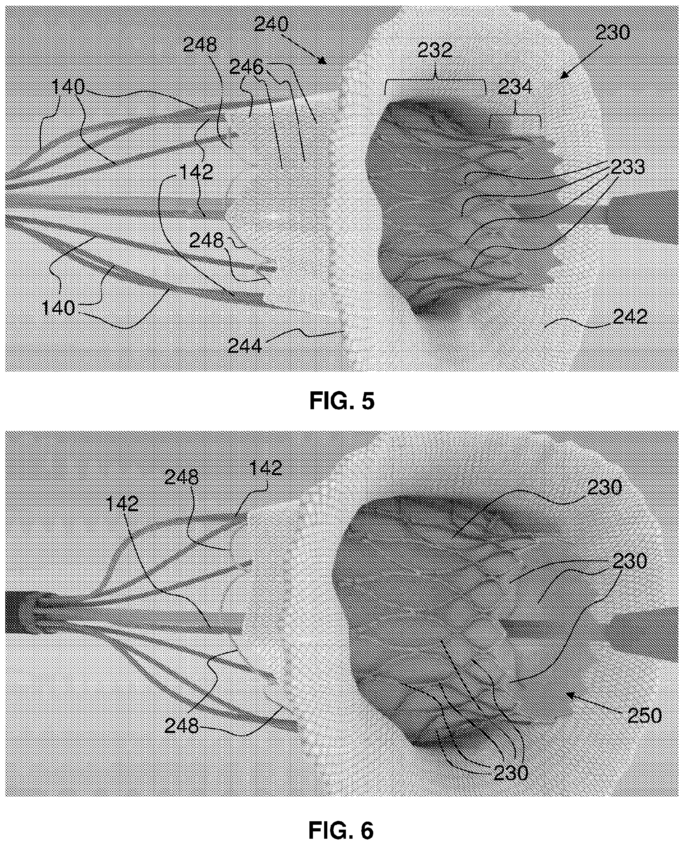

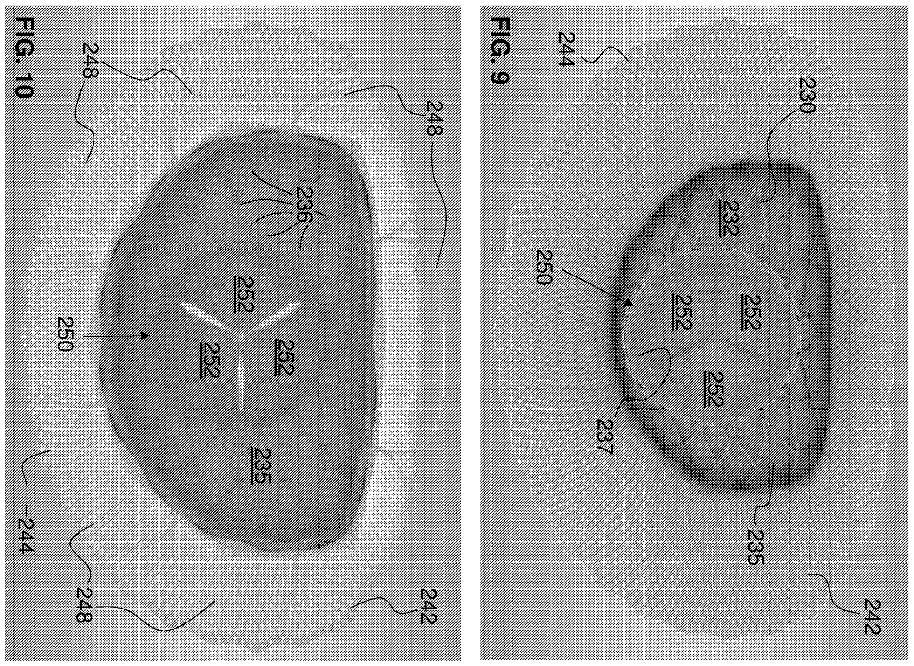

The implant 200 has an external, adjustable stent lattice 210. The stent lattice 210 can be of a shape memory material (such as nitinol, for example). The adjustable stent lattice 210 is set to a pre-determined shape that, in this exemplary mitral valve embodiment, is D-shaped as shown in FIG. 5 and, particularly, in FIGS. 9 to 12. (In an alternative exemplary embodiment where the implant site is circular, the adjustable stent lattice would be pre-set to a circular shape.) Connected to the interior of the adjustable stent lattice 210 is a self-expanding valve trampoline lattice 230. The trampoline lattice 230 can be integral with the adjustable stent lattice 210 or fixedly connected thereto, for example, by crimping, banding, welding. A set of hollow disconnect lumens 140 surround lattice drive wires 150 and both are operatively connected to a non-illustrated delivery and deployment handle (also referred to as a controller because it need not be shaped as a handle). The exemplary embodiment of the disconnect lumens 140 are tubes that extend from the handle to the implant 200 and each surround a drive wire 150. Lattice disconnect tubes 142 are respectively disposed at the end of each hollow disconnect lumens 140. The lattice disconnect tubes 142 are rotationally fixed to the disconnect lumens 140 such that, when the disconnect lumen 140 rotates, the respective lattice disconnect tube 142 rotates correspondingly. The lattice disconnect tubes 142 surround the distal end of the drive wires 150, at which distal end are drive wire connectors 152. The drive wire connectors 152 are each shaped to connect to respective proximal ends of jack screws 220 that are rotatably connected to the adjustable stent lattice 210 as described in further detail below.

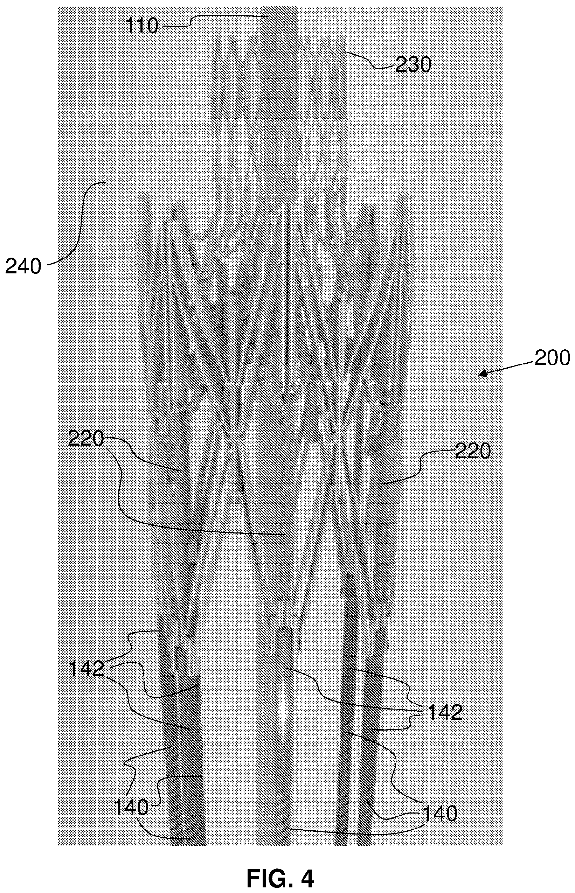

FIG. 2 depicts the implant 200 without an implant skirt and a valve trampoline (which are removed for clarity but are shown in FIG. 4). The implant 200 is in a pre-installation orientation before the drive wires 150 have caused the jack screws 220 to expand the adjustable stent lattice 210. The lattice disconnect tubes 142 at each distal end of the disconnect lumens 140 are partially visible from under the outer sheath 130. Under the lattice disconnect tubes 142, the drive wires 150 are removably connected to the proximal end of each jack screw 220 so that, as long as the lattice disconnect tubes 142 remain in this distally disposed state adjacent the proximal end of the adjustable stent lattice 210, the drive wires 150 are rotationally fixed to the proximal end of each jack screw 220, which means that, as a drive wire 150 rotates, the respective jack screw 220 rotates correspondingly. The non-illustrated delivery and deployment handle controls expansion and contraction of the adjustable stent lattice 210 and deployment of the implant 200 by rotation of the drive wires 150 and, when deployment is desired, by proximal movement of the disconnect lumens 140, which, when moved proximally, translate the lattice disconnect tubes 142 away from the connection between the drive wires 150 and the expansion/contraction assembly of the adjustable stent lattice 210 (see jack screws below) to permit automatic separation of the drive wires 150 and the jack screws.

The self-expanding valve trampoline lattice 230 is disposed within a central orifice of the adjustable stent lattice 210 and is attached to the adjustable stent lattice 210. FIG. 3 shows the sheath 130 entirely removed from the implant 200 and the lattice disconnect tubes 142. Here, the adjustable stent lattice 210 is further expanded towards its pre-defined self-expanding shape. Visible in FIG. 3 is the connection location of the drive wire connectors 152 and the proximal end of the jack screws 220, but that connection is hidden within the respective lattice disconnect tubes 142.

FIG. 4 illustrates the implant 200 in approximately its fully self-expanded state. The exterior implant skirt 240 and the valve trampoline lattice 230 are shown but are transparent in this figure. As will be explained in further detail below, the implant skirt 240 is attached (e.g., with sutures in the shape of an "X") to the exterior surfaces of the adjustable stent lattice 210.

FIG. 5 illustrates the implant 200 in a partially forcibly expanded state and FIG. 6 illustrates the implant 200 in a further expanded state. The implant 200 is rotated in FIG. 6 to show more of the left ventricle side of the implant, i.e., the side of the implant configured to be positioned with the left ventricle of the patient. Here, the exterior implant skirt 240 and the valve trampoline lattice 230 are fully resolved and, therefore, the implant skirt 240 covers the adjustable stent lattice 210 so that it is no longer visible. The self-expanding, memory shaped skirt lattice 242 is visible beneath the exterior material 244 of the implant skirt 240 for clarity. The material 244 can be made from anything that is fluid-tight or resistant and retains that fluid-tight or resistant characteristic even after being attached to the skirt lattice 242 or to the adjustable stent lattice 210 with sutures that puncture the material 244, for example. The material 244 can be a woven polyester fabric, a sheet of plastic, and/or a sheet of pericardial tissue, for example. Preferably, the material 244 is of braided polyester coated with polyurethane. As can be seen on the exterior surface of the material 244, sutures 246 (e.g., in the shape of an "X") fixedly attach the material 244 and the skirt lattice 242 to the adjustable stent lattice 210 therein such that, as the adjustable stent lattice 210 expands, both the skirt lattice 242 and the material 244 expands correspondingly, as is shown, for example, in the transition from FIG. 5 to FIG. 6.

Extending proximally from skirt lattice 242 (and being part of the skirt lattice 242) are left-atrium wall-retaining petals 248. Even though the material 244 prevents viewing of the entire extent of the retaining petals 248, it can be seen in FIGS. 5 and 6 that the lattice disconnect tubes 142 are disposed radially outside the retaining petals 248 but inside the skirt lattice 242/material 244. In this manner, the petals 248 are prevented from expanding radially outwards until the lattice disconnect tubes 142 are disconnected from the adjustable stent lattice 210. FIG. 6 shows the implant 200 in an exemplary fully-expanded, delivery orientation with the drive wires 150 still engaged to the implant 200. It is noted that the circular shape of the retaining petals 248 is only one exemplary configuration for retaining the implant 200 on the atrium side of the mitral valve annulus. These petals 248 can take any shape that, after being allowed to pivot in a direction from the guidewire lumen 110 radially outward, allows the implant 200 to be secured on the atrium side.

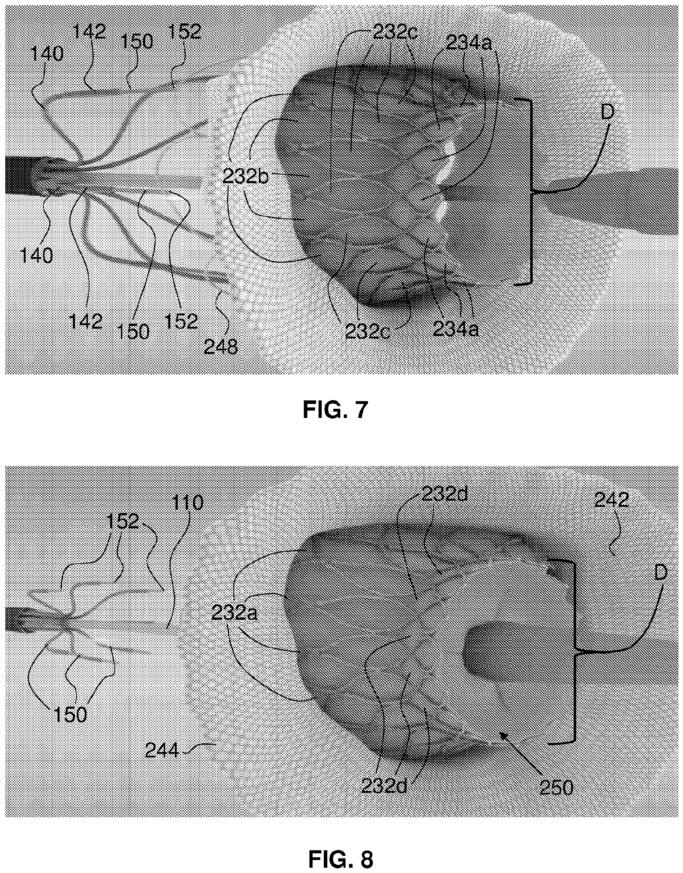

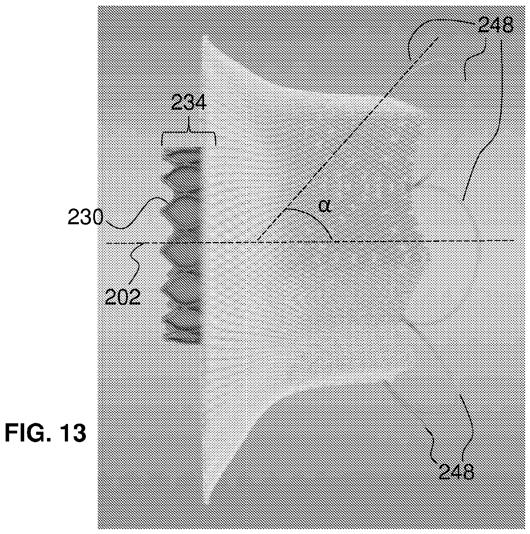

When the implant 200 is positioned in a final desired orientation, such as that shown in FIGS. 7 through 13, the drive wires 150 are to be disconnected from all of the jack screws 220 and, thereafter, removed from the patient. To disengage the drive wires 150, a non-illustrated control handle moves each of the disconnect lumens 140 proximally (either simultaneously or separately) to thereby slide the hollow disconnect tubes 142 away from the adjustable stent lattice 210. When this covering is removed, the temporary connection of the drive wire connectors 152 and the proximal ends of the jack screws 220 can be allowed to separate from one another, as shown in FIG. 7, allowing the disconnect lumens 140 and the drive wires 150 (and the sheath 130 if desired) to retract away from the adjustable stent lattice 210. The collapsed or contracted state of the wall-retaining petals 248 only remains while the disconnect lumens 140 and the drive wires 150 remain connected to the adjustable stent lattice 210. When disconnected therefrom, the petals 248 are allowed to move to their steady state or pre-set orientation, which is a position where the petals 248 extend in a plane that is substantially perpendicular to the axis of the guidewire lumen 110. Likewise, when allowed to self-expand, the skirt lattice 242 moves to its steady-state or pre-set orientation, which is a position where the edges of the skirt lattice 242 extend in a plane that is more perpendicular to the axis of the guidewire lumen 110 than parallel thereto. This orientation of the implant 200 secured in a target location, such as the mitral valve annulus, is depicted in the views of FIGS. 9 to 12 and, in particular, in the side elevational view of FIG. 13. As can be seen in FIG. 13, the angle α between the petals 248 and the central axis 202 of the implant 200 is between approximately 45 degrees and approximately 90 degrees including every number therebetween. In particular, the angle α is between approximately 50 degrees and approximately 75 degrees including every number therebetween.

Also shown in FIGS. 5 to 13 is the structure of the valve trampoline lattice 230, including a centrally disposed valve 250, which, in this exemplary embodiment, is a replacement for a mitral valve. More particularly, as shown in FIG. 5, the self-expanding valve trampoline lattice 230 has an expandable outer trampoline portion 232 and an expandable but fixed maximum diameter inner circumferential valve portion 234, which can be cylindrical and, for example, fabricated from a laser-cut, nitinol tube. The outer trampoline portion 232 connects on its exterior circumference to an interior circumference of the adjustable stent lattice 210. The connection can be integral or can be formed, for example, by crimping, banding, and/or welding. Deformable cells of the outer trampoline portion 232 allow the outer trampoline portion 232 to expand and contract substantially. In this example, the cells are substantially marquis-shaped or tear-drop shaped, but they can take other closed curved or straight shapes. More particularly in the exemplary embodiment of FIGS. 5 to 13, the valve trampoline lattice 230 has figure-eight-shaped structures that, when attached together at their sides, form five rows of cells that circumscribe the valve trampoline lattice. The outer trampoline portion 232 includes the first four rows of tear-drop and marquis-shaped cells. A first row of relatively smaller tear-drop-shaped cells 232a (see FIGS. 7 and 8) are sixteen in number and define an outermost ring of outer trampoline portion 232 cells. A second row of relatively larger marquis-shaped cells 232b, also sixteen in number, define a first inner ring of the outer trampoline portion 232. A third row of sixteen marquis-shaped cells 232c narrower and longer than the cells 232b define a second inner ring of the outer trampoline portion 232. Finally, a fourth row of sixteen smallest marquis-shaped cells 232d define a third inner ring of the outer trampoline portion 232. In comparison, the inner circumferential valve portion 234 of the valve trampoline lattice 230 has one row of sixteen cells 234a about its circumference, each cell having a longitudinal length that is about the same as the circumferential width. Accordingly, these sixteen cells 234a are substantially circular, even though they have tear-drop tips on either end. The substantially circular nature of the sixteen cells 234a creates a cylinder at the interior end of the valve trampoline lattice 230. This compound structure allows the inner circumferential valve portion 234 to remain substantially circular even while the outer trampoline portion 232 expands and contracts with expansion or contraction of the adjustable stent lattice 210.

Accordingly, when the adjustable stent lattice 210 expands or contracts, the outer circumference of the outer trampoline portion 232 correspondingly expands or contracts without limitation. The inner circumferential valve portion 234 connects to the outer trampoline portion 232 at cell connection points 233. This inner circumferential valve portion 234 is not D-shaped and does not have cells that allow it to expand and contract in the same way that the cells of the outer trampoline portion 232 permit unrestricted expansion. Instead, in this exemplary configuration, the cells of the inner circumferential valve portion 234 only allow expansion up to a pre-determined state once the adjustable stent lattice 210 is expanded far enough to no longer constrain the inner circumferential valve portion 234. At that state, which is shown starting at FIG. 6, the cells forming the outer circumference of the inner circumferential valve portion 234 are disposed about the central axis 202 of the implant 200 in a substantially circular manner defining a pre-determined maximum diameter D, shown in FIGS. 7 and 8 and continues in FIGS. 9 to 13. Thus, no matter how far the adjustable stent lattice 210 expands the outer trampoline portion 232 of the trampoline lattice 230, the inner circumferential valve portion 234 will not expand past the diameter D.

The reason why the trampoline lattice 230 is referred to as a "trampoline" is because of the way that it supports the valve 250. At the inner circumferential valve portion 234, the trampoline lattice 230 is substantially constant after the adjustable stent lattice 210 has expanded to no longer restrict the inner circumferential valve portion 234. The outer trampoline portion 232, in contrast, expands to whatever shape is needed to bridge the gap between the inner circumferential valve portion 234 and the adjustable stent lattice 210. Thus, the outer portion 232 acts as a stretchable "trampoline" to move and adjust to whatever shape is needed to suspend the relatively stable inner circumferential valve portion 234 (and the valve 250) at the central region of the trampoline lattice 230. The natural shape of the outer trampoline portion 232 corresponds to the inner circumference of the adjustable stent lattice 210, which means it has a natural D-shaped circumference.

Both the outer trampoline portion 232 and the inner circumferential valve portion 234 are fluid-tightly sealed to the material 244 so that, when installed, the implant 200 forms a fluid-tight seal that only permits fluid flow through the valve 250. As shown best in FIGS. 9 and 10, a first sealing material 235 is secured to the inside surfaces of the outer trampoline portion 232 and a second sealing valve material 237 is secured to the inside surfaces of the inner circumferential valve portion 234. The second sealing valve material 237 can be a single sheet with three leaflets cut therein or it can be a set of three separate leaflet portions individually connected to the interior surfaces of the inner circumferential valve portion 234. The material 235 can be a woven polyester fabric, a sheet of plastic, and/or a sheet of pericardial tissue, for example. The material 237 can be pericardial tissue or a natural valve harvested from a mammal, such as a porcine valve. In this exemplary embodiment, the second sealing valve material 237 extends from the distal most ends of the inner circumferential valve portion 234 to the center of the valve 250 and, thereby, forms the leaflets 252 of the valve 250, which, in this embodiment, is a tricuspid form (i.e., three leaflets 252). The tricuspid form is not to be considered as limiting and can have any number of leaflets. The first sealing material 235 and the second sealing valve material 237 can be separate, with the leaflets 252 extending into the central orifice of the inner circumferential valve portion 234 from either the first or second materials 235, 237, or they can be integral, with the leaflets 252 being a part of the material portions 235, 237 and extending into the central orifice of the inner circumferential valve portion 234.

With the petals 248 on the atrium side of the now-installed implant 200 and the skirt lattice 242 with its material 244 on the ventricle side of the implant 200, the diseased mitral valve annulus is captured and surrounded by the implant 200 in a liquid-tight and leak-free manner. Viewed in a cross-sectional plane extending along the axis of the guidewire lumen 110, therefore, the petals 248 and the skirts lattice 242 with the material 244 forms a U-shaped annular raceway as depicted in FIG. 13.

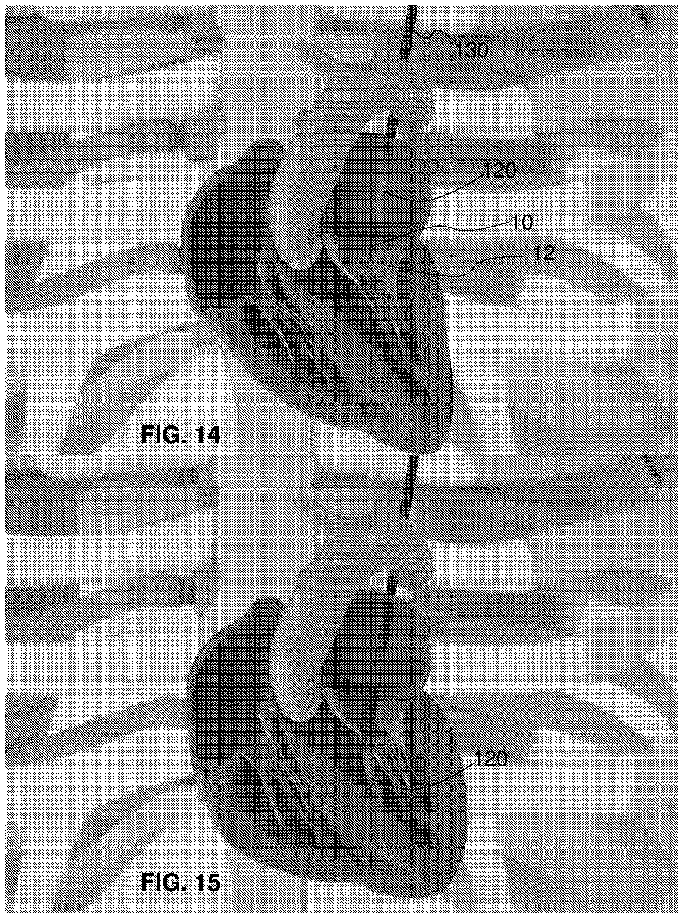

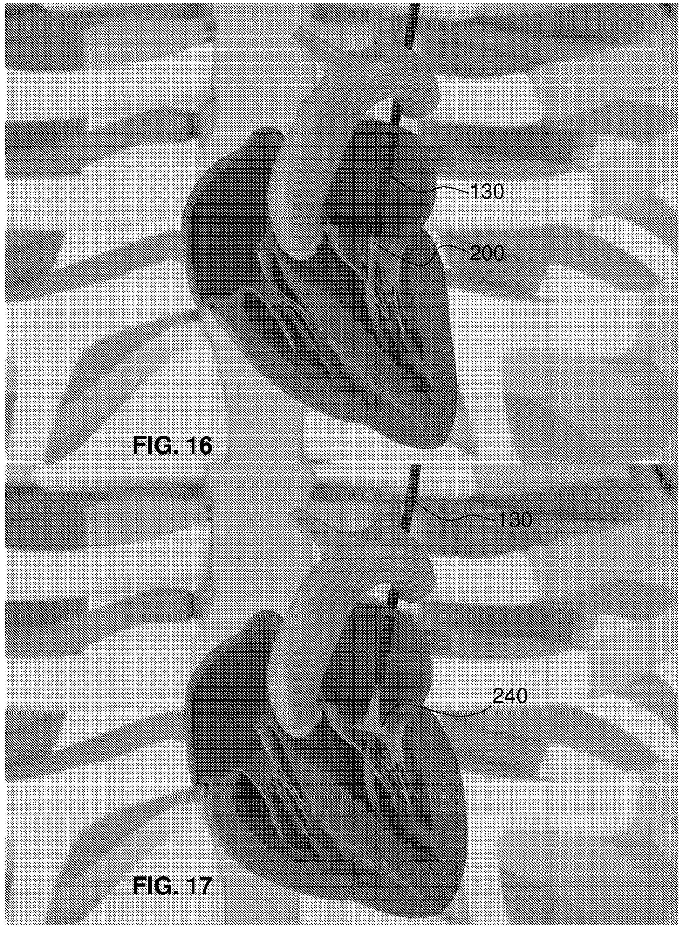

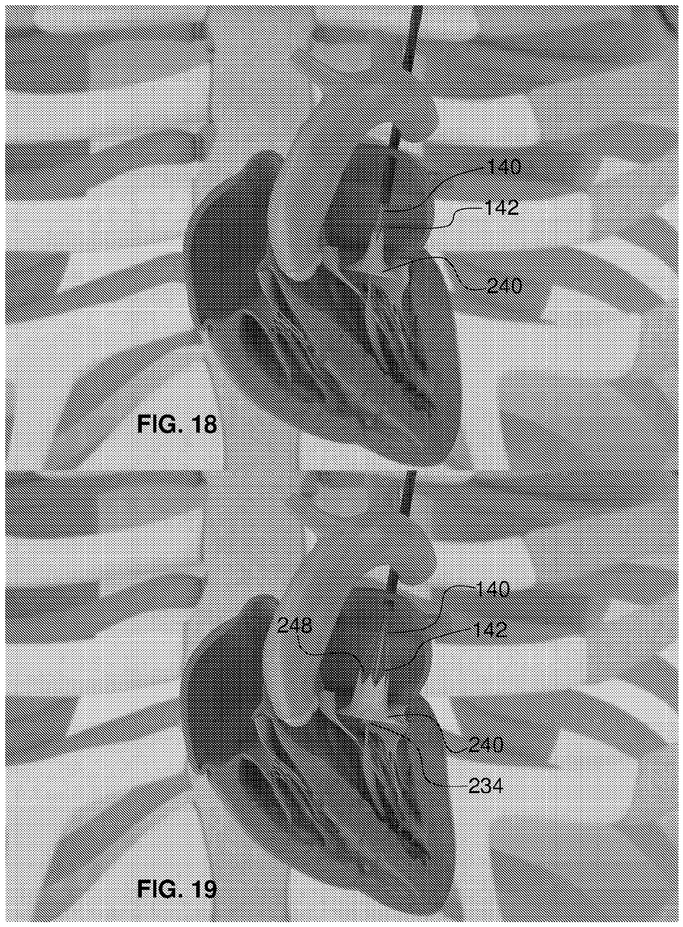

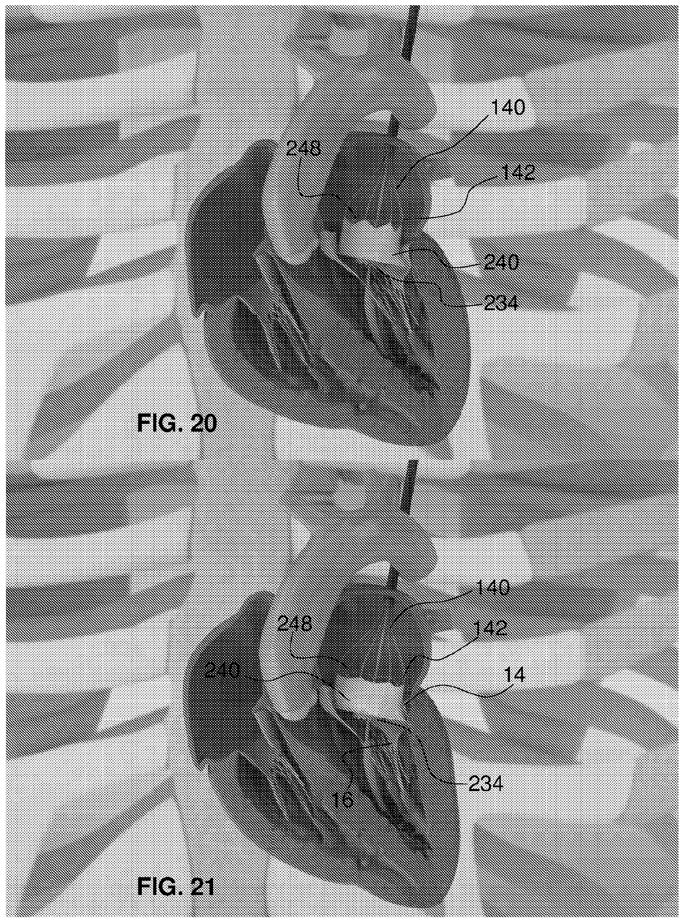

This capture of the native mitral valve annulus is depicted in the progression of FIGS. 14 to 22, which is an example of replacement mitral valve implantation with a transapical approach. In FIG. 14, a guidewire 10 has been installed through the atrium wall and the diseased mitral valve 12 and rests in the left ventricle. The guidewire lumen 110 is threaded onto the guidewire 10 and the guidewire lumen 110 enters the left atrium preceded by the distally connected nosecone 120. The nosecone 120 of the delivery system 100 is extended into the left ventricle in FIG. 15. In FIG. 16, the outer sheath 130 has been withdrawn into the left atrium and the mitral valve replacement implant 200 contained within the sheath 130 starts to be exposed within the diseased mitral valve annulus. In FIG. 17, the outer sheath 130 has been withdrawn almost all of the way over the mitral valve replacement implant 200 to an extent where the implant skirt 240 is able to self-expand into and towards the sub-valvular structures below the diseased mitral valve annulus. In FIG. 18, the outer sheath 130 has been withdrawn completely from the mitral valve replacement implant 200 to an extent where the sheath 130 only constrains the disconnect lumens 140 and drive wires 150. The implant skirt 240 is further self-expanded into the sub-valvular structures below the diseased mitral valve annulus. In FIG. 19, the outer sheath 130 has been withdrawn completely to no longer radially constrain the disconnect lumens 140 or drive wires 150 to, thereby, allow the adjustable stent lattice 210 to self-expand to its pre-determined, memory shape. The implant skirt 240 is further self-expanded into and touching the sub-valvular structures below the diseased mitral valve annulus to an extent that it could reach and touch the chords beyond the native leaflet edges if sized appropriately. FIG. 20 shows the adjustable stent lattice 210 forcibly expanded radially outwards and the implant skirt 240 remaining secured against the sub-valvular structures below the diseased mitral valve annulus but also adapting to the forcibly expanded orientation of the internal adjustable stent lattice 210. Finally, FIG. 21 illustrates the mitral valve replacement implant 200 in a fully expanded and implanted orientation within the mitral valve annulus just before disconnection of the drive wires 150. As can be seen, the implant skirt 240 substantially engages the native leaflets 14 and chordae tendineae 16 to create a fluid tight seal at the mitral valve annulus.

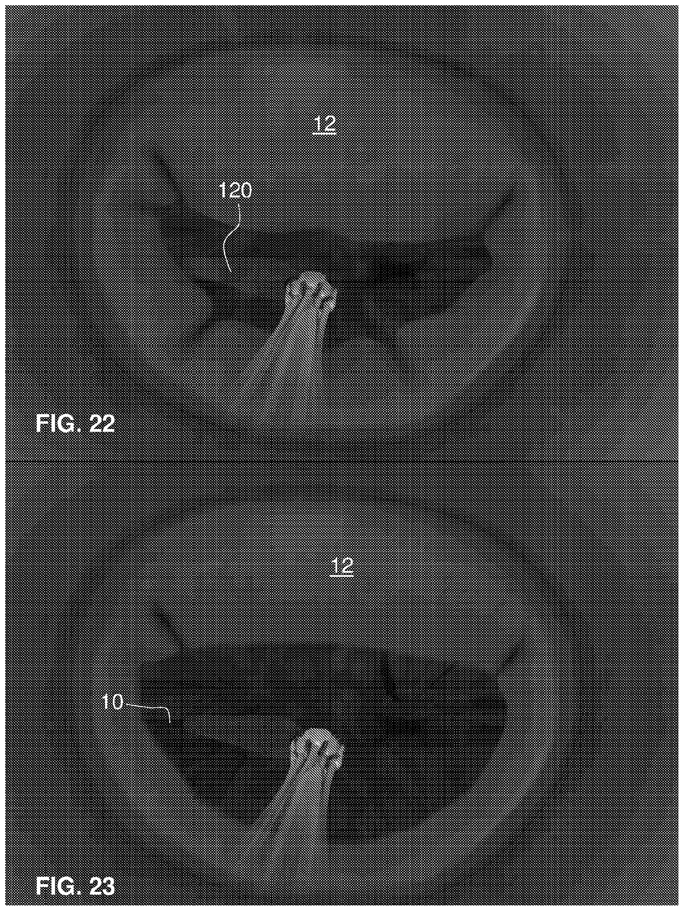

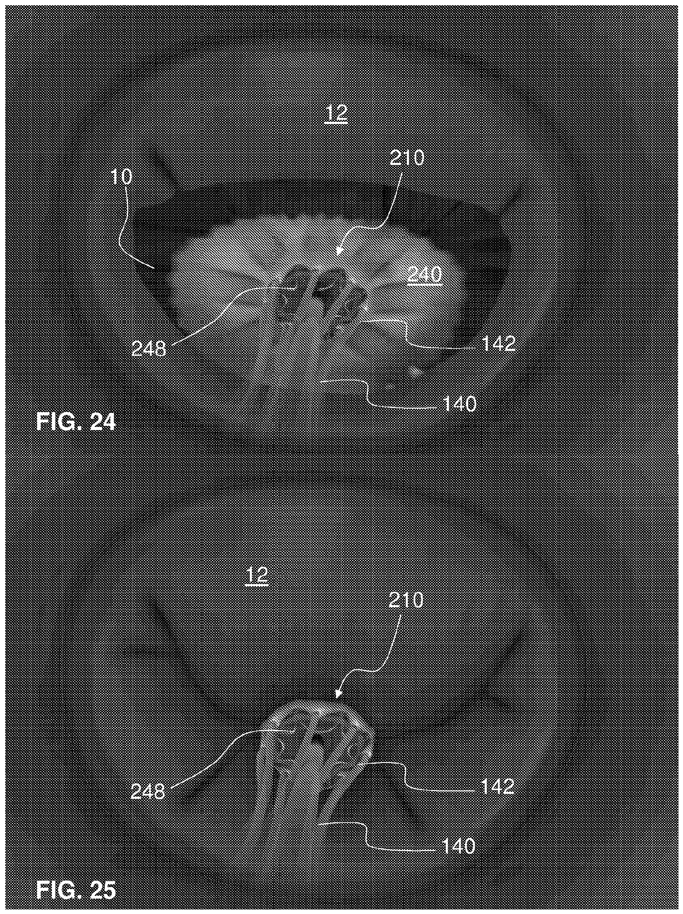

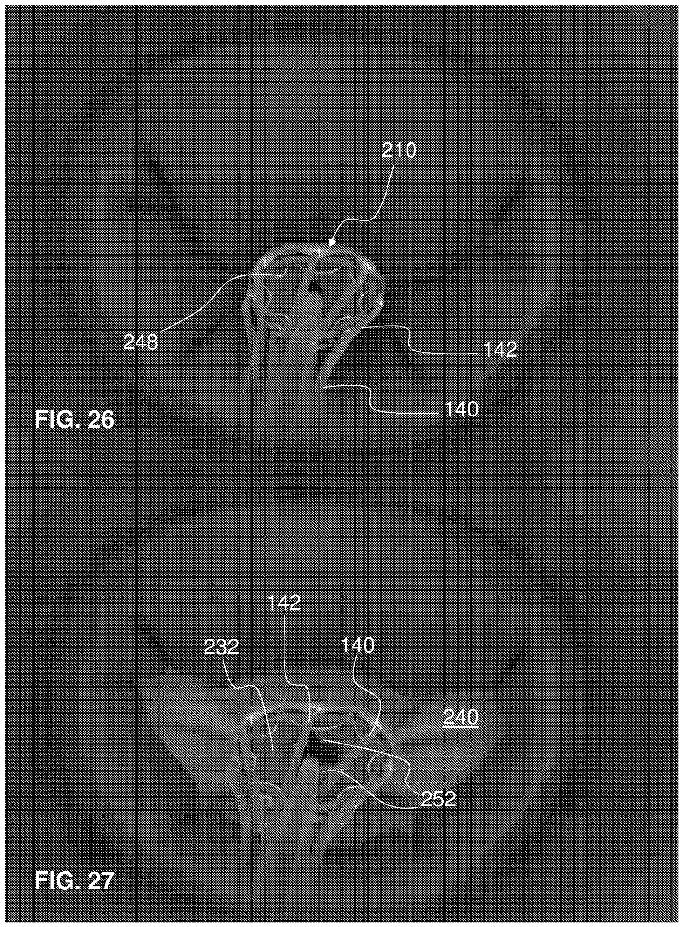

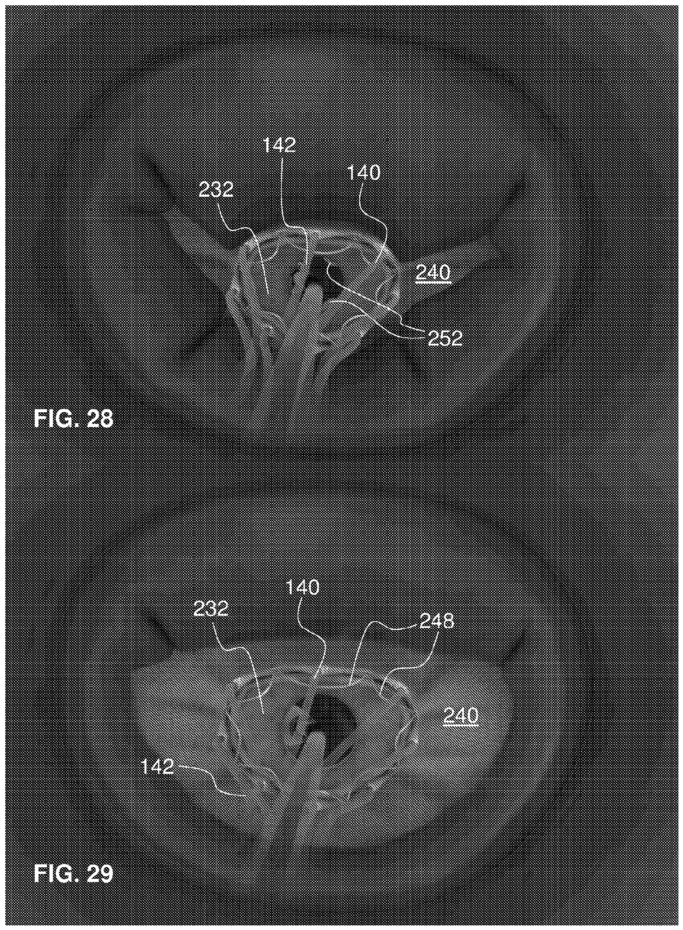

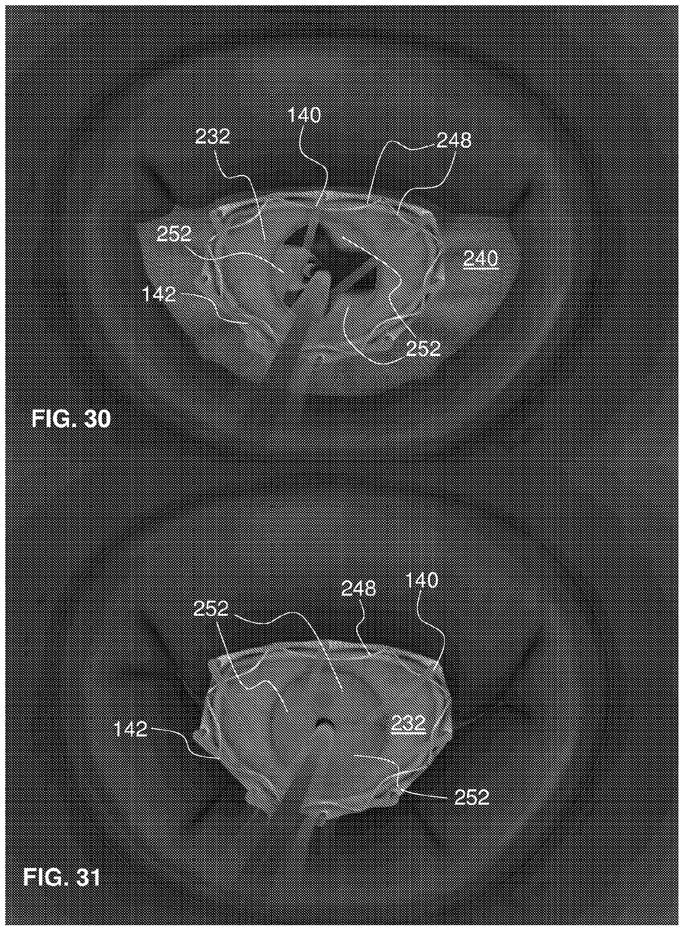

The process for installing the implant 240 from the atrial side is shown in the progression of FIGS. 22 through 35. FIGS. 22 and 23 illustrate the guidewire 10 resting in the left ventricle (installed through the atrium wall) and the diseased mitral valve 12. The guidewire lumen 110 is threaded onto the guidewire 10 and enters the left atrium preceded by the distally connected nosecone 120, resting in the left ventricle. The mitral valve 12 is shown partially closed in FIG. 22 and substantially open in FIG. 23. The implant 200 is compressed within the exterior sheath 130 but the sheath 130 is not illustrated for clarity. In FIG. 24, the outer sheath 130 has been retracted to allow the implant 240 to start self-expanding to its pre-defined, mitral valve D-shape and the implant skirt 240 to start self-expanding within the ventricle to its pre-defined memory shape. In this figure, the mitral valve 12 is substantially open. The adjustable stent lattice 210 is positioned within the mitral valve annulus, as can be seen in FIG. 25, where the mitral valve 12 is closed upon the adjustable stent lattice 210. As the drive wires 150 and disconnect lumens 140 are still connected to the adjustable stent lattice 210, the atrium wall-retaining petals 248 are constrained at the interior sides of the disconnect lumens 140. With the implant 200 centrally disposed within the mitral valve 12, the drive wires 150 can be rotated to actuate the jack screws 220 and, thereby, start expansion of the implant 200. FIG. 26 shows the implant 200 in the state where the implant 200 is fully self-expanded and defines the pre-defined D-shape of the implant (albeit smaller than when implanted), and FIG. 27 shows the implant 200 in a first, forcibly expanded state, with the D-shape larger than the pre-set D-shape. With the mitral valve 12 partially open around the implant 200, the implant skirt 240 can be seen on the ventricle side self-expanded to such an extent that it already occludes the opening of the mitral valve 12, which is due both to the self-expanding features of the implant skirt 240, 242, 244 and to the pressure exerted on the ventricle side of the implant skirt 240 by blood flow. Likewise, this blood flow imparts a force on the replacement valve 250, causing the leaflets 252 to open and allow blood flow through the implant 200. With still further expansion of the implant 200, as shown in FIGS. 28 and 29, expansion of the outer trampoline portion 232 becomes more apparent and the replacement valve 250 begins to function as a valve well before full implantation occurs. This valve functioning is clearly shown in FIGS. 30 and 31, in which, a further expanded state of the implant 200 allows the leaflets 252 of the replacement valve 250 to fully open and fully close (about the guidewire lumen 110).

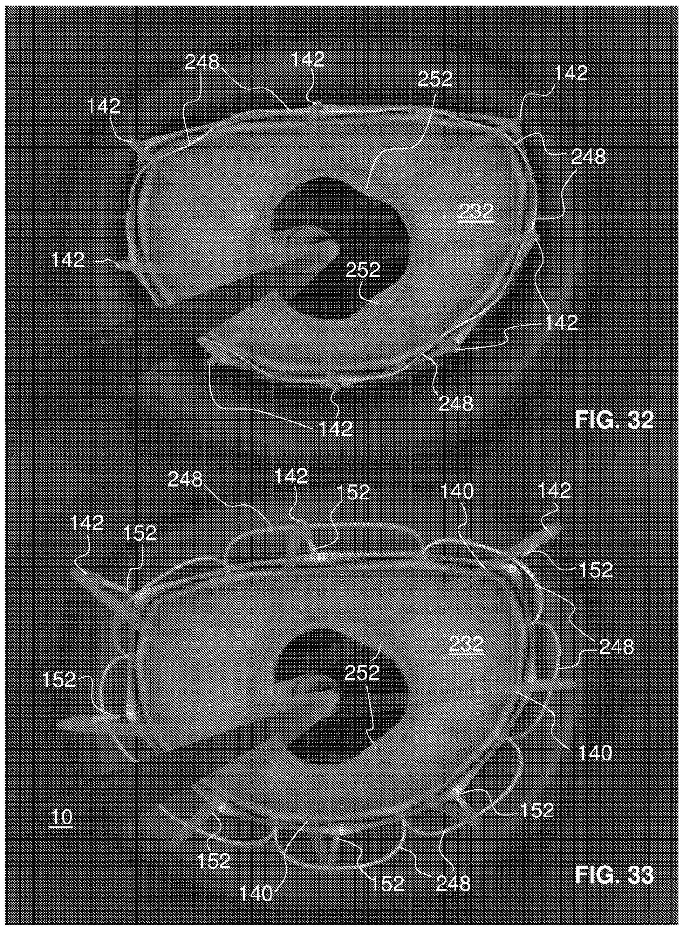

When the implant 200 has been expanded to a state where it is fixed in the diseased mitral valve 12 to hold the diseased mitral valve 12 open, as shown in FIG. 32, the implant 200 is ready to be disengaged from the delivery system 100. At this point, the surgeon can ensure correct implantation because the implant 200 has the ability to reversibly contract and be repositioned if desired. To confirm that the implant 200 is in a desired final state, the surgeon can inject a contrast dye into the ventricle (e.g., through the guidewire lumen 110 or through the guidewire 10, if either is configured to deliver dye, or through a separate contrast device within the ventricle). When the surgeon has determined that no leakage occurs around the implant 200 and that the implant 200 is satisfactorily installed, the surgeon can actuate the disengagement feature of the delivery system 100, which, in this exemplary embodiment, causes the disconnect lumens 140 to retract proximally and, thereby, move the lattice disconnect tubes 142 away from the drive wire connectors 152. FIG. 33 illustrates the disconnect lumens 140 having retracted the lattice disconnect tubes 142 from the drive wires 150 to expose the drive wire connectors 152 (which engage the proximal ends of the jack screws 220 and remain engaged only so long as the lattice disconnect tubes 142 are extended) and, thereby, allow the drive wire connectors 152 to disengage automatically from the jack screws 220. At this point, implantation is final and cannot be reversed without physically removing the implant 200, e.g., through a secondary open surgical procedure. However, such a situation is not necessary when the surgeon has ensured correct placement before disengagement.

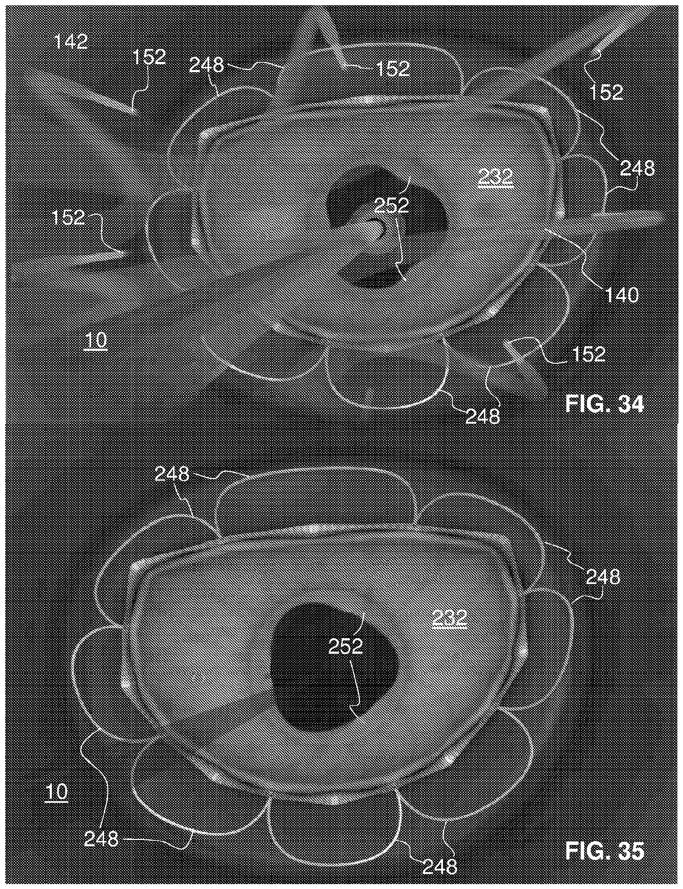

Now that the disconnect lumens 140 and lattice disconnect tubes 142 no longer restrain the atrium wall-retaining petals 248, the petals 248 can expand outward to their pre-set orientation. FIG. 33 shows the petals 248 in the process of this expansion and FIG. 34 illustrates the petals 248 fully expanded and compressing the ventricle side of the mitral valve 12 in a direction of the implant skirt 240, which, itself, is fully expanded and imparting a compressive force against the atrium side of the mitral valve 12. As the implant process is complete, the disconnect lumens 140 and the drive wires 150 can be removed, which is depicted in FIG. 35.

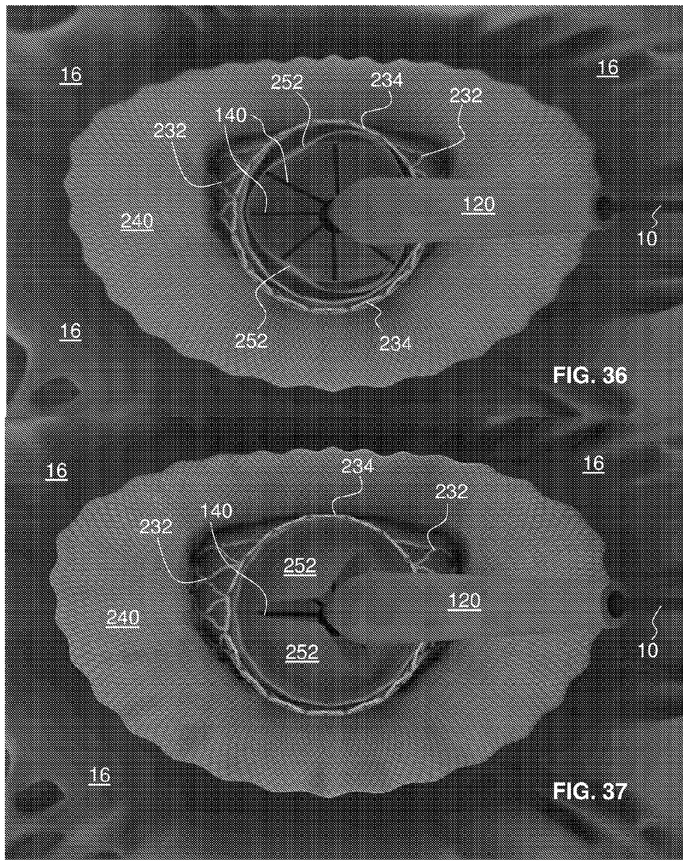

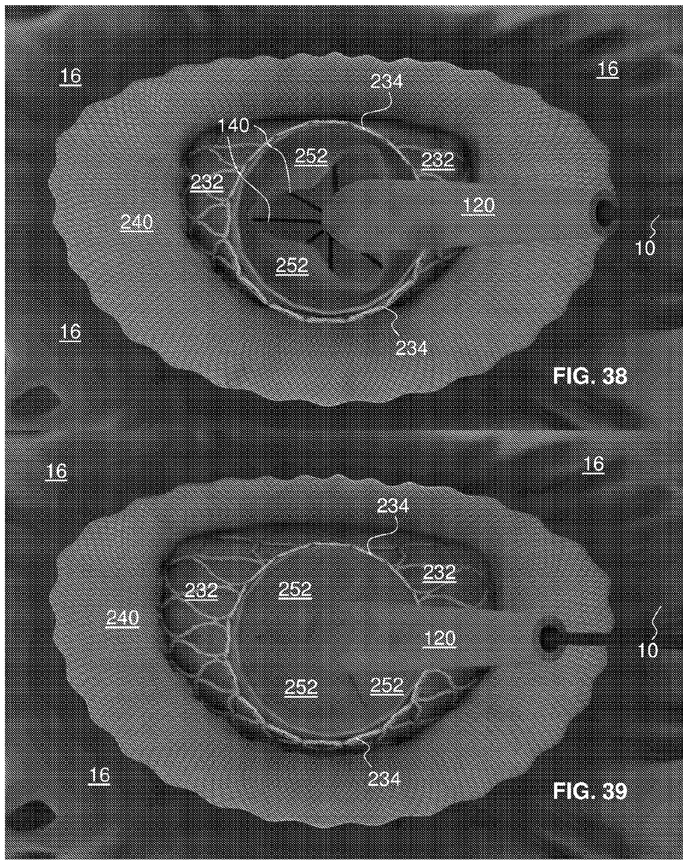

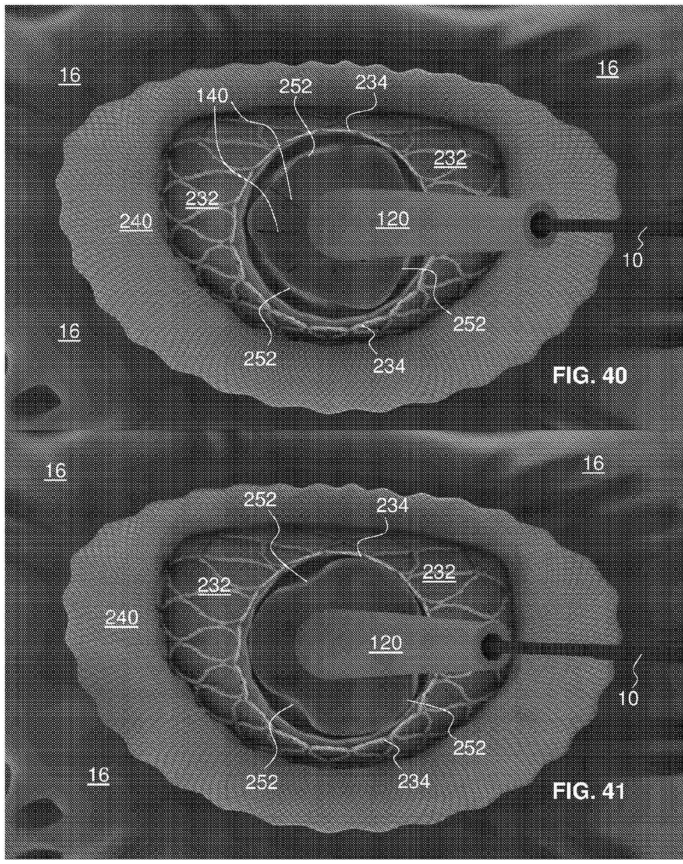

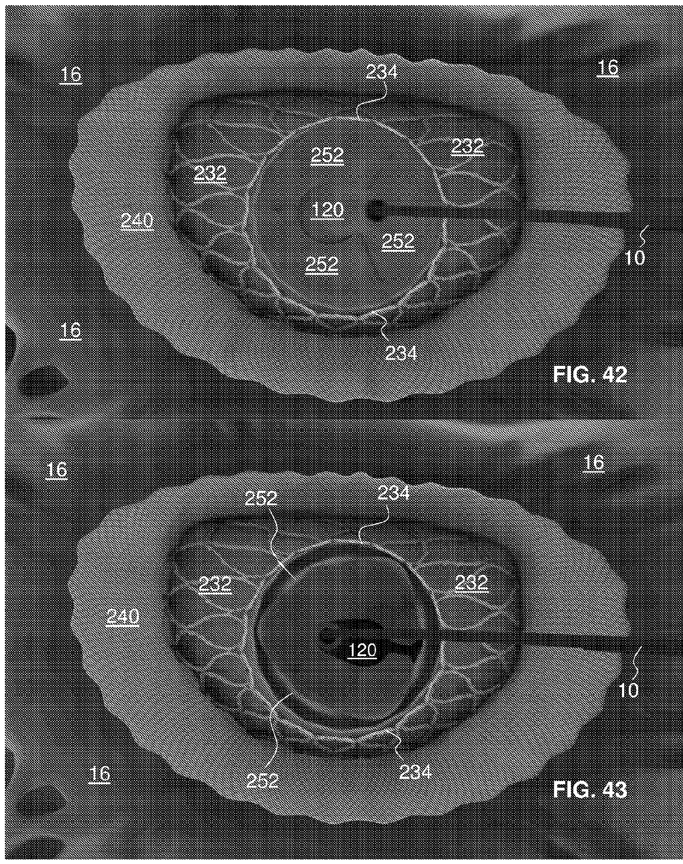

Above, the implant skirt 240 is described as self-expanding on the ventricle side of the mitral valve 12 starting from the time that it is released from capture within the exterior sheath 130. That expansion is shown from a side of the implant 240 in FIGS. 18 through 21, 24, and 27. FIGS. 36 through 43, however, show the behavior of the implant skirt 240 as the adjustable stent lattice 210 is forcibly expanded from its fully self-expanded state (shown in FIG. 36) until the time of final implantation (shown in FIG. 40). FIGS. 36 through 43 show this behavior viewed from the ventricle side of the implant 200. As the adjustable stent lattice 210 forcibly expands from its self-expanded state in FIG. 36 through the range of expansion in FIGS. 37, 38, and 39, it can be seen that the innermost portions of the implant skirt 240, including the skirt lattice 242 and the outer material 244, move and adjust to accommodate the ever-expanding outer extremities of the outer trampoline portion 232. In contrast, the outermost annulus of the implant skirt 240 remains substantially constant from the time that it is completely released from the sheath 130 until the time when the implant 200 is in its final implant orientation, which is seen in FIG. 40. At this point, the stent control devices (disconnect lumens 140 and drive wires 150) can be disconnected and withdrawn, which is depicted in FIG. 40, these devices 140, 150 being entirely removed from view in FIG. 41 and the nosecone 120 being withdrawn in the progression of FIG. 42 to FIG. 43. These figures illustrated that the replacement valve 250 has functioning leaflets 252 from the time that the adjustable stent lattice 210 is merely allowed to self-expand and continues functioning as a valve all during the time that the implant 200 is being adjusted, expanded, contracted, moved, and/or rotated. These figures also show how the valve trampoline lattice acts as a trampoline. The inner circumferential valve portion 234 remains patent in its circular orientation throughout the time that the outer trampoline portion 232 is being expanded (or contracted).

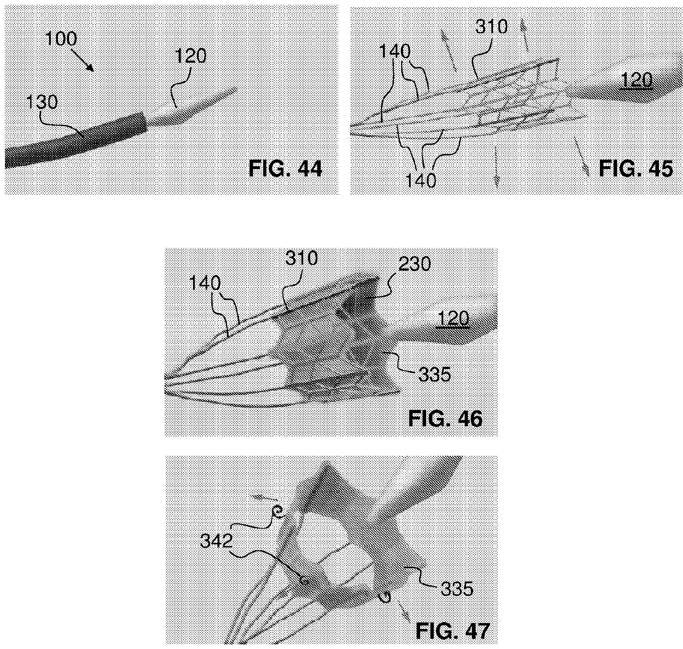

FIGS. 44 to 47 illustrate an alternative embodiment of an actively controllable stent graft 300. This implant is similar in structure to the implant 200 except the wall-retaining petals 248 and the exterior implant skirt 240 are not present. Although the interior valve assembly also is not present, any valve assembly can be included within the adjustable stent lattice 310 of the implant 300. The implant 300 can be substantially circular or it can be D-shaped. As all other aspects of the implant 300 are similar to the implant 200, all features are not repeated to avoid unnecessary repetition. Likewise, where similar parts are referenced, the reference numeral is increased by 100. What is different with the implant 300 is that the tissue-fixation structures are extendible hooks 342 that are part of or attached to the adjustable stent lattice 310. Such hooks 342 can take the form of the needles 1700 or 2200 or 3070 shown and described in U.S. Patent Publication No. 2013/0046373 to Cartledge et al.

Deployment of the implant 300 is performed just as implant 200. The delivery system 100 in FIG. 44 is guided along a non-illustrated guidewire to an implant site in the patient. The adjustable stent lattice 310 is allowed to self-expand after removal of the sheath 130 and then is forcibly expanded into the annulus of the implant site to a final implant size, the implant 300 being reversibly expanded and contracted as desired to achieve optimal deployment. This expansion is shown in FIGS. 45 and 46 (the graft material 335 of the implant 300 is not illustrated in FIG. 45). When the implant 300 is in the desired location and orientation and is ready to be released, the extendible hooks 342 are extended out from the sides of the implant 300 to enter into and fixedly connect to the tissue at the implant site. Only three of the hooks 342 are illustrated in FIG. 47 but this number is not to be limiting. In an exemplary embodiment, the number of hooks 342 is equal to the number of disconnect lumens 140, which in the embodiment shown would be six in number.

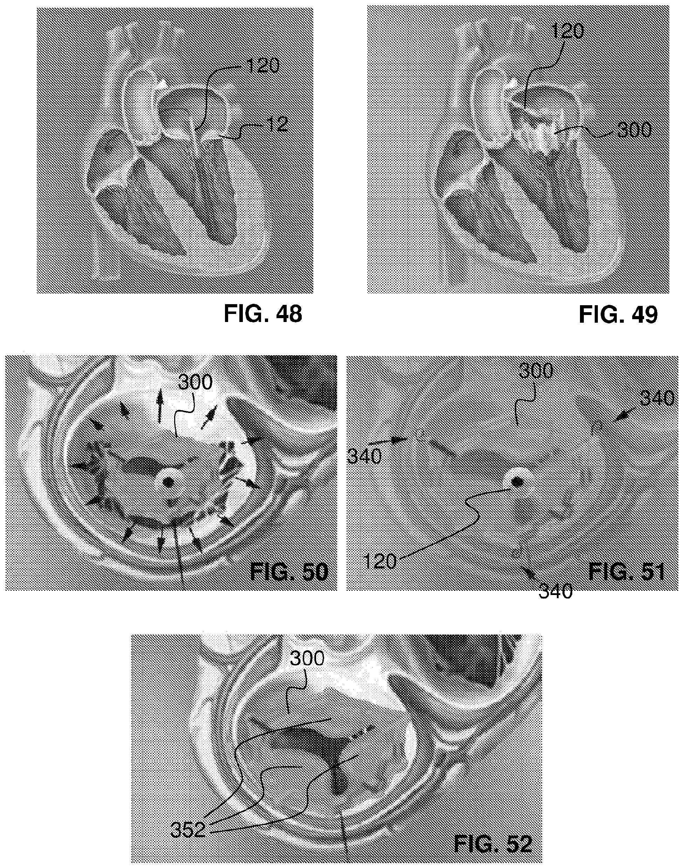

FIGS. 48 to 52 illustrate the process for deploying the implant 300, for example, in the mitral valve of a heart. In contrast to the embodiment of implant 200, which is inserted through the atrium from above the heart, the implant 300 is inserted through a transapical approach. The delivery system 100 is inserted through the apex of the heart up through the left ventricle and through the mitral valve 12 to place the nosecone 120 within the left atrium. The implant 300 is expanded into the mitral valve 12 as shown in FIGS. 49 and 50. When ready to implant, the hooks 342 are extended into the wall of the heart. In FIG. 52, the implant 300 is released from the delivery system 100 and the internal valve leaflets function to valve the blood flow.

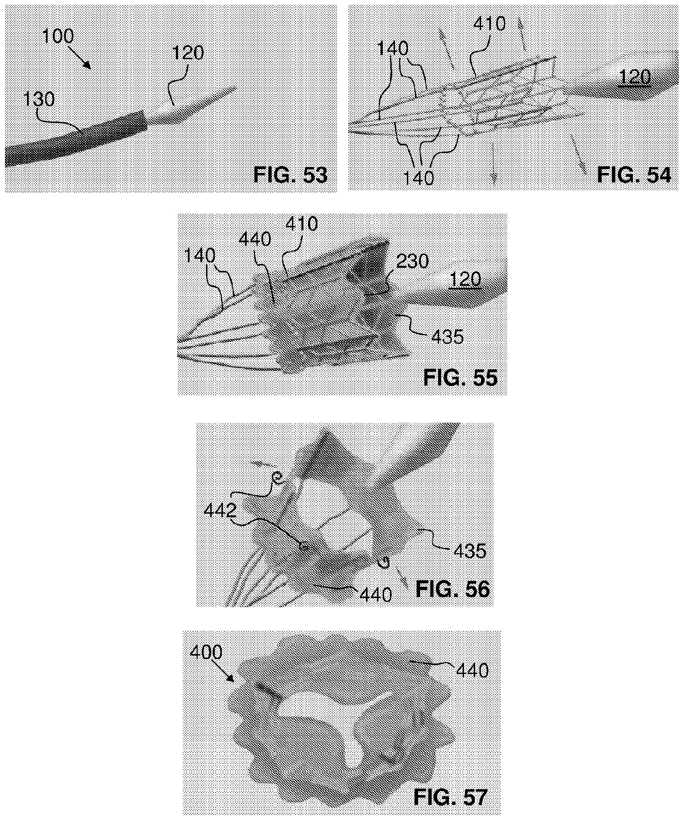

FIGS. 53 to 57 illustrate an alternative embodiment of an actively controllable replacement mitral valve implant 400. This implant is similar in structure to the implant 200 except the wall-retaining petals 248 are not present. Like implant 200, the implant 400 has an exterior implant skirt 440. The implant 400 can be substantially circular, but it is D-shaped in this mitral valve embodiment. As all other aspects of the implant 400 are similar to the implant 200, all features are not repeated to avoid unnecessary repetition. Likewise, where similar parts are referenced, the reference numeral is increased by 200. What is different with the implant 400 is that the implant skirt 440 is supplemented with tissue-fixation structures in the form of extendible hooks 442 that are part of or attached to the adjustable stent lattice 410. Such hooks 442 can take the form of the needles 1700 or 2200 or 3070 shown and described in U.S. Patent Publication No. 2013/0046373 to Cartledge et al.

Deployment of the implant 400 is performed just as implant 200. The delivery system 100 in FIG. 53 is guided along a non-illustrated guidewire to an implant site in the patient. The adjustable stent lattice 410 is allowed to self-expand after the sheath is removed and then is forcibly expanded into the annulus of the mitral valve to a final implant size, the implant 400 being reversibly expanded and contracted as desired to achieve optimal deployment. As with implant 200, when the sheath 130 is removed from the compressed implant 400, the implant skirt 440 is allowed to self-expand on the ventricle side of the mitral valve annulus. This expansion is shown in FIGS. 54 and 55 (the graft material 435 of the implant 400 is not illustrated in FIG. 54). When the implant 400 is in the desired location and orientation and is ready to be released, the extendible hooks 442 are extended out from the sides of the implant 400 to enter into and fixedly connect to the tissue at the implant site. Only three of the hooks 442 are illustrated in FIG. 56 but this number is not to be limiting. In an exemplary embodiment, the number of hooks 442 is equal to the number of disconnect lumens 140, which in the embodiment shown would be six in number.

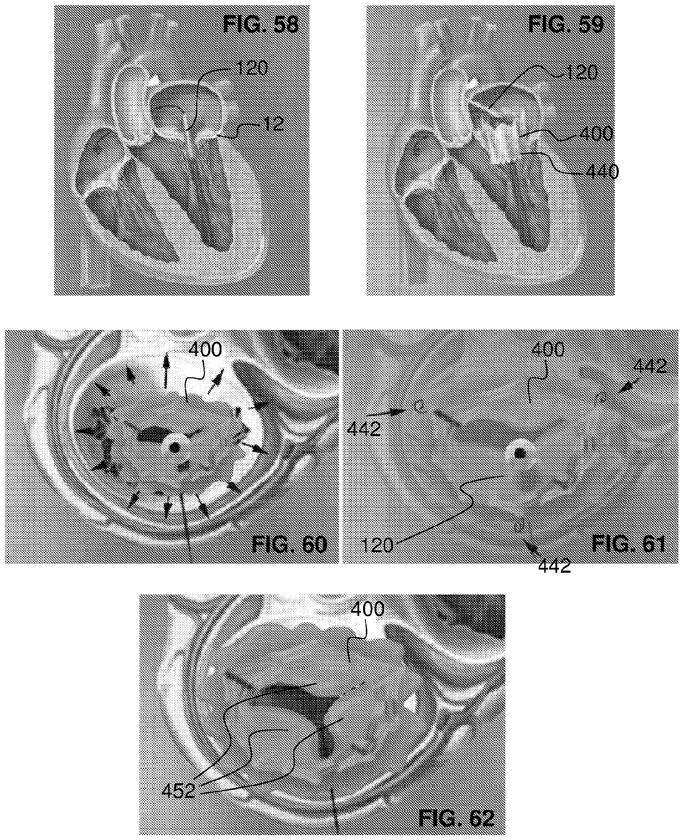

FIGS. 58 to 62 illustrate the process for deploying the implant 400 in the mitral valve annulus of a heart. In contrast to the embodiment of implant 200, which is inserted through the atrium from above the heart, the implant 400 is inserted through a transapical approach. The delivery system 100 is inserted through the apex of the heart up through the left ventricle and through the mitral valve 12 to place the nosecone 120 within the left atrium. The implant 400 is expanded into the mitral valve 12 as shown in FIGS. 59 and 60. When ready to implant, the hooks 442 are extended into the wall of the heart. In FIG. 62, the implant 400 is released from the delivery system 100 and the internal valve leaflets 452 function to valve the blood flow.

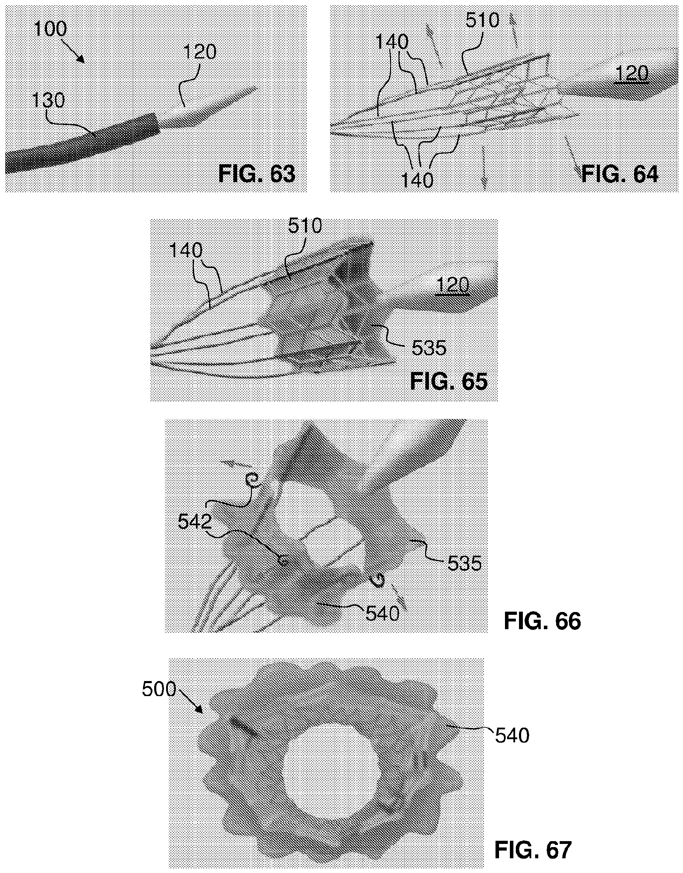

FIGS. 63 to 67 illustrate an alternative embodiment of an actively controllable replacement mitral valve implant 500. This implant is similar in structure to the implant 200 except the wall-retaining petals 248 are not present. Like implant 200, the implant 500 has an exterior implant skirt 540. The implant 500 can be substantially circular, but it is D-shaped in this mitral valve embodiment. As all other aspects of the implant 500 are similar to the implant 200, all features are not repeated to avoid unnecessary repetition. Likewise, where similar parts are referenced, the reference numeral is increased by 300. What is different with the implant 500 is that the implant skirt 540 is supplemented with tissue-fixation structures in the form of extendible hooks 542 that are part of or attached to the adjustable stent lattice 410. The self-expanding valve trampoline lattice 530 is also present. The hooks 542 can take the form of the needles 1700 or 2200 or 3070 shown and described in U.S. Patent Publication No. 2013/0046373 to Cartledge et al.

Deployment of the implant 500 is performed just as implant 200. The delivery system 100 in FIG. 63 is guided along a non-illustrated guidewire to an implant site in the patient. The adjustable stent lattice 510 is allowed to self-expand after the sheath is removed and then is forcibly expanded into the annulus of the mitral valve to a final implant size, the implant 500 being reversibly expanded and contracted as desired to achieve optimal deployment. As with implant 200, when the sheath 130 is removed from the compressed implant 500, the implant skirt 540 is allowed to self-expand on the ventricle side of the mitral valve annulus. This expansion is shown in FIGS. 64 and 65 (the graft material 535 of the implant 500 is not illustrated in FIG. 64). When the implant 600 is in the desired location and orientation and is ready to be released, the extendible hooks 542 are extended out from the sides of the implant 500 to enter into and fixedly connect to the tissue at the implant site. Only three of the hooks 542 are illustrated in FIG. 66 but this number is not to be limiting. In an exemplary embodiment, the number of hooks 542 is equal to the number of disconnect lumens 140, which in the embodiment shown would be six in number.

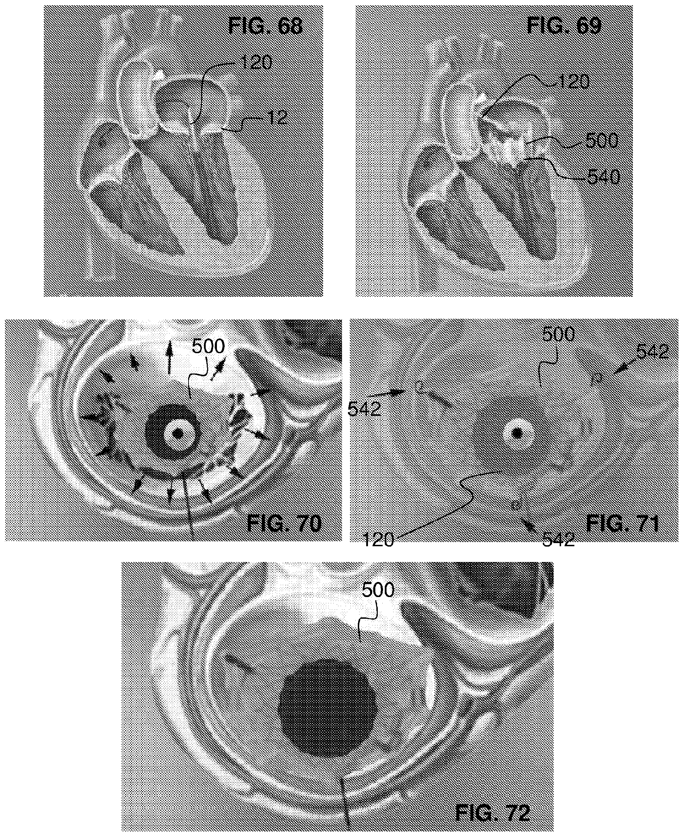

FIGS. 68 to 72 illustrate the process for deploying the implant 500 in the mitral valve annulus of a heart. In contrast to the embodiment of implant 200, which is inserted through the atrium from above the heart, the implant 500 is inserted through a transapical approach. The delivery system 100 is inserted through the apex of the heart up through the left ventricle and through the mitral valve 12 to place the nosecone 120 within the left atrium. The implant 500 is expanded into the mitral valve 12 as shown in FIGS. 69 and 70. When ready to implant, the hooks 542 are extended into the wall of the heart. In FIG. 72, the implant 500 is released from the delivery system 100 and the internal, non-illustrated valve leaflets function to valve the blood flow.

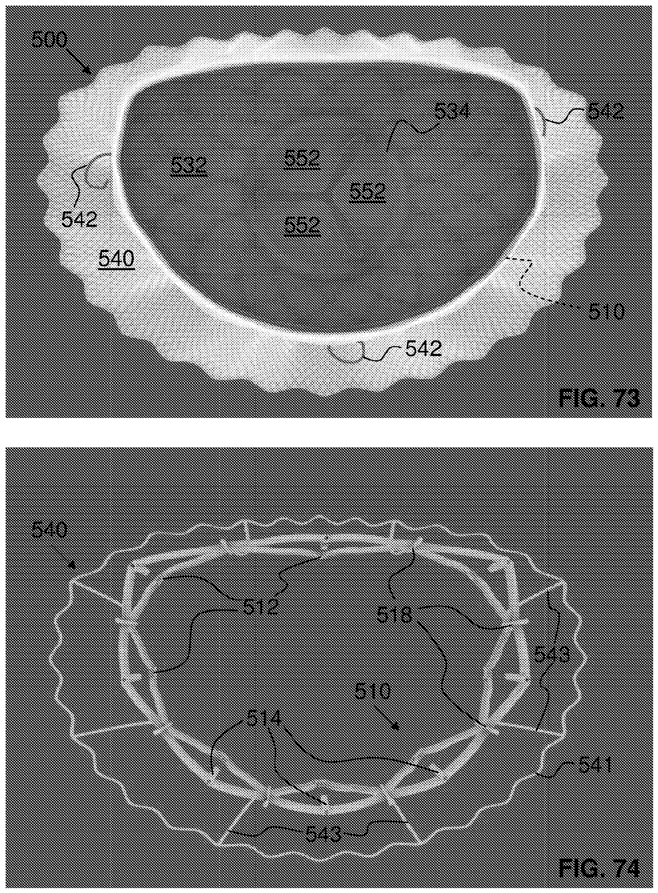

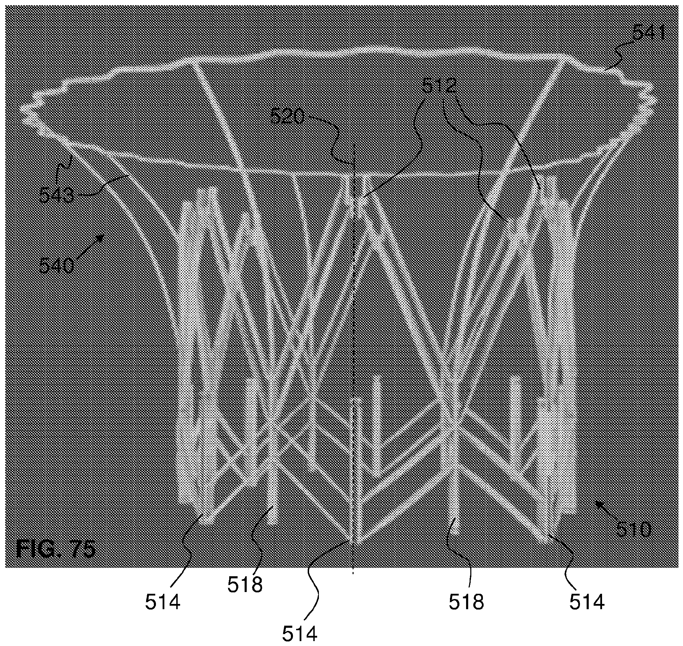

FIG. 73 illustrates an exemplary embodiment of the implant 500, viewed from the atrium side thereof with the expandable outer trampoline portion 532 deployed and the leaflets 552 of the inner circumferential valve portion 534 in an almost closed state. FIGS. 74 and 75 show the skeleton of the implant 500 with the outer material and trampoline valve removed. The implant skirt 540 comprises two parts, an outer circumferential ring 541 and a set of ring-connecting struts 543, which connect the ring 541 to the adjustable stent lattice 510. Both the ring 541 and the struts 543 are made of a material that is self-expanding and having a desired, pre-set shape (such as heat-set nitinol, for example). The struts 543 can be integral with the ring 541 and/or the adjustable stent lattice 510 or connected thereto. The various parts of the adjustable stent lattice 510 are best viewed in FIG. 75. The adjustable stent lattice 510 comprises sets of jack screw connectors, each set having a proximal jack strut 512 and a distal jack strut 514. Arms 516 connect each of the proximal and distal jack struts 512, 514 to an intermediate strut 518. Jack screws 520, which are non-illustrated for clarity but one is depicted as a dashed line in FIG. 75, connect to the proximal and distal jack struts 512, 514 so that, when the jack screw 520 is turned in one direction, the proximal and distal jack struts 512, 514 separate from one another and, when the jack screw 520 is turned in the other opposite direction, the proximal and distal jack struts 512, 514 move towards one another. This configuration can be achieved in various ways. One exemplary embodiment fixes a non-illustrated threaded nut within the distal jack strut 514 to allow the jack screw 520 to threadedly enter into and retract from a smooth-bored hollow in the distal jack strut 514 and places a rotationally free but longitudinally fixed connection of the jack screw 520 at the proximal jack strut 512. In this manner, when the jack screw 520 is rotated in a strut-approaching direction, the distal end of the jack screw 520 moves into the internal non-threaded bore of the distal jack strut 514 (via the connection with threads of the nut) and the proximal end of the jack screw 520 remains longitudinally fixed at the proximal jack strut 512 but is allowed to rotate freely therein. As set forth above, the proximal end of the jack screw 520 has a connector part that is removably connected to a drive wire connector 150 of a drive wire 150 so that, when the drive wire connector 150 is caused to rotate, the jack screw 520 rotates correspondingly. This connection is maintained in any of the exemplary embodiments when the lattice disconnect tubes surround both the proximal end of the jack screw (referred to as a driving connector) and the drive wire connector 150. One way to form the removable connection is through a form fit, such as two cylinders having a mirrored handshake keying that only remains connected when a hollow cylindrical shroud encircles that connection. A form-locking or form-fitting connection is one that connects two elements together due to the shape of the elements themselves, as opposed to a force-locking connection, which locks the elements together by force external to the elements.

With such a configuration, rotation of the many jack screws 520 in the strut-approaching direction causes the proximal and distal jack struts 512, 514 to move towards one another and, thereby, push the intermediate struts 518 (which are disposed parallel to the jack screws 520) away from the jack screw 520 in a direction along the circumferential extent of the annulus of the adjustable stent lattice 510. This relative movement of the intermediate strut 518 and the jack screw assemblies causes expansion of the adjustable stent lattice 510 when the proximal and distal jack struts 512, 514 move towards one another and causes contraction of the adjustable stent lattice 510 when the proximal and distal jack struts 512, 514 move away from one another. Ideally, all of the jack screws 520 are rotated at the same speed to but such equal movement is not to be considered limiting.

In the exemplary embodiment of the adjustable stent lattice 510 shown, there are eight pairs of jack struts 512, 514 and eight intermediate struts 518. This number is merely exemplary and there can be, for example, only six of each or any other number desired including any number from 1 to 10. Connecting the pairs of jack struts 512, 514 and the intermediate struts 518 are the laterally extending arms 516. As the adjustable stent lattice 510 is either contracted or expanded, the arms 516 each flex at their two endpoints, one at a respective intermediate strut 518 and the other at a respective one of a pair of jack struts 512, 514. As can be seen from the configuration shown in FIG. 75, when the adjustable stent lattice 510 is contracted (e.g., for installation into the delivery sheath 130), the arms 516 move towards a longitudinal orientation (parallel to the jack screws and to the central axis of the lattice 510. Conversely, when the adjustable stent lattice 510 is expanded (e.g., for implantation), the arms 516 angle away from the respective intermediate strut 518 and one of the pair of jack struts 512, 514 in a circumferential orientation (perpendicular to the jack screws).

While this detailed description of the parts of the adjustable stent lattice 510 is present herein with respect to implant 500, it is equally applicable to the each of the alternative implant embodiments described herein.

As stated above, the structures forming the various exemplary embodiments for heart valves are not limited to only a single valve or a single exterior shape. The features can be extended to alternative configurations.

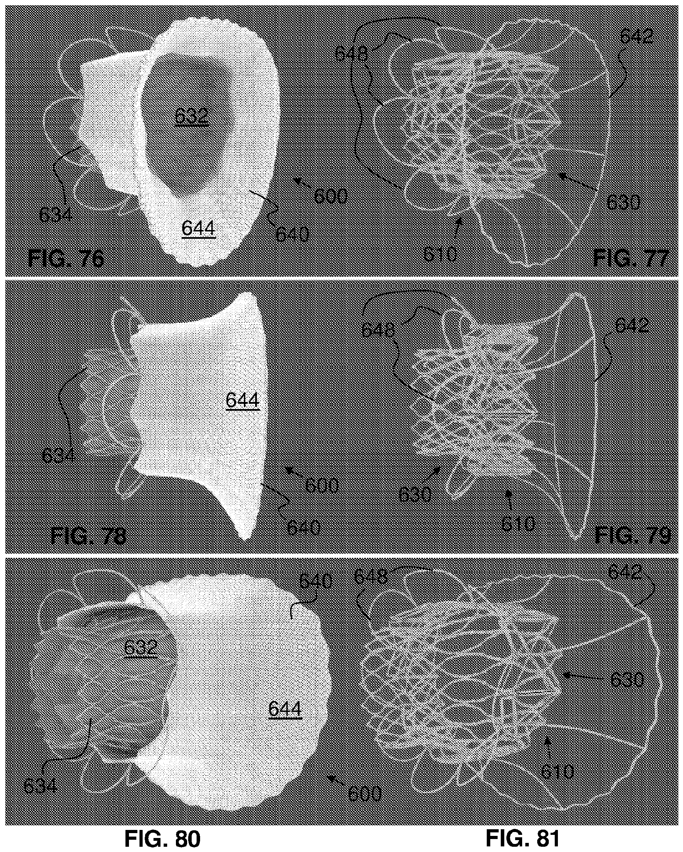

A first alternative configuration of a mitral valve replacement implant 600 is shown in FIGS. 78 to 81. In the embodiment of FIGS. 2 to 13, the mitral valve replacement implant 200 had the trampoline lattice 230 projecting through the implant skirt 240 on the ventricle side of the implant 200. The exemplary embodiment of the mitral valve replacement implant 600 in FIGS. 78 to 81 provides the trampoline lattice 630 with the inner circumferential valve portion 634 on the opposing side and projecting out from the side of the implant 600 with the wall-retaining petals 648. The expandable outer trampoline portion 632 is visible in FIG. 76. The framework of the implant 600 is shown without the external coverings and valve material in FIGS. 77, 79, and 81 to expose the adjustable stent lattice 610, the skirt lattice 642, and the wall-retaining petals 648.

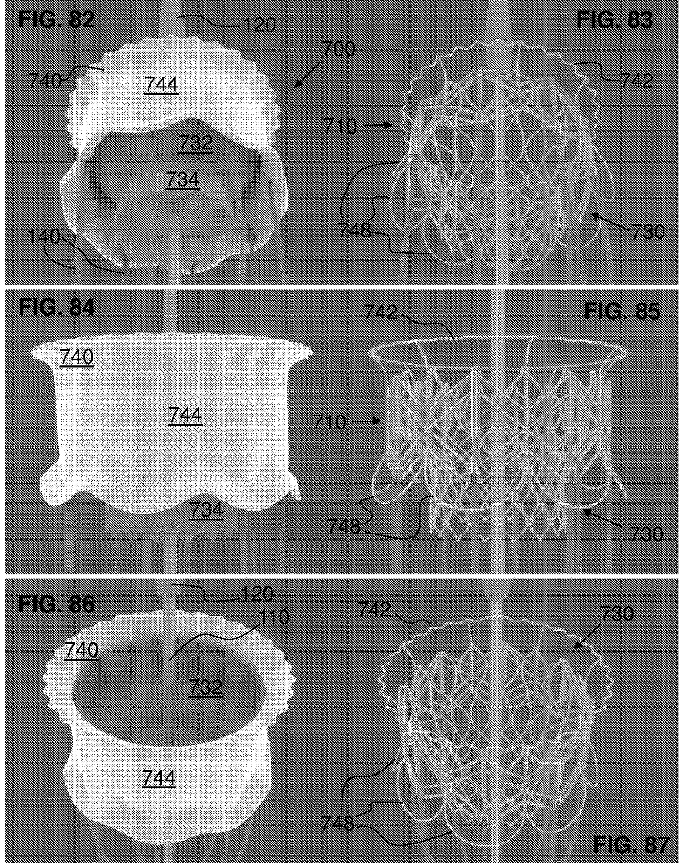

A second alternative configuration of a circular valve replacement implant 600 is shown in FIGS. 82 to 87. In the embodiment of FIGS. 78 to 81, the implant 600 had an overall D-shape and the wall-retaining petals 648 were uncovered. The exemplary embodiment of the valve replacement implant 700 in FIGS. 82 to 87 likewise provides the trampoline lattice 730 with the inner circumferential valve portion 734 on the side opposite the implant skirt 740 projecting out from the side of the implant 700 with the wall-retaining petals 748 but is circular in its overall shape. Both the expandable outer trampoline portion 732 and the inner circumferential valve portion 734 are visible in FIG. 82. As can be seen, the material 744 of the implant skirt 740 also covers the wall-retaining petals 748. The framework of the implant 700 is shown without the external coverings and valve material in FIGS. 83, 85, and 87 to expose the adjustable stent lattice 710, the skirt lattice 742, and the wall-retaining petals 748. Because the disconnect lumens 140 and the drive wires 150 connect from the side of the wall-retaining petals 748, the nosecone 120 is on the side of the implant skirt 740.

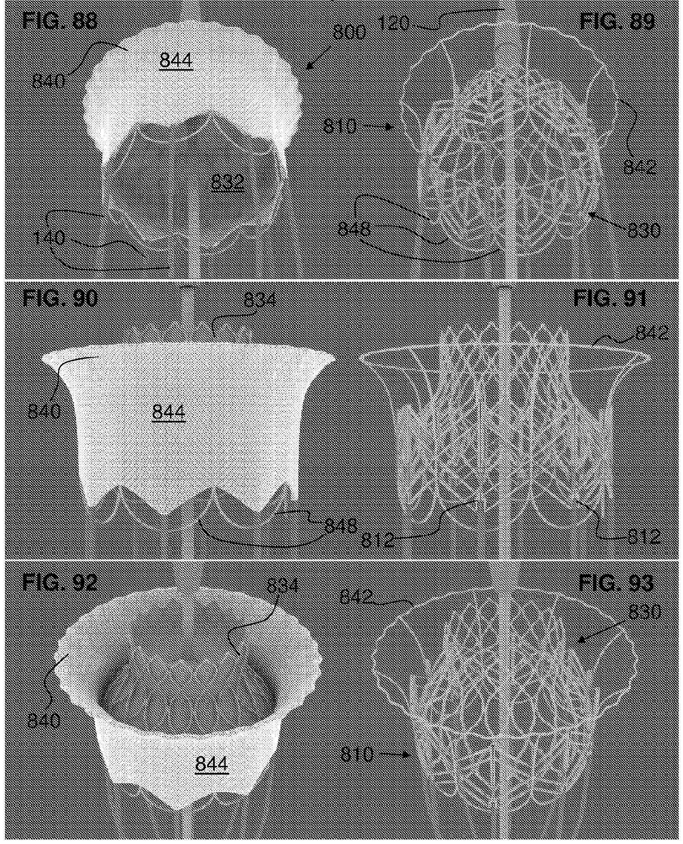

A third alternative configuration of a circular valve replacement implant 800 is shown in FIGS. 88 to 93. In the embodiment of FIGS. 82 to 87, the implant 700 had an overall circular shape and the wall-retaining petals 748 were covered. Like the implant 700, the implant 800 is circular in its overall shape. In contrast to the implant 700, however, the exemplary embodiment of the valve replacement implant 800 in FIGS. 88 to 93 has the trampoline lattice 830 with the inner circumferential valve portion 834 on the side opposite the wall-retaining petals 848 to project out from the side of the implant 800 having the implant skirt 840. The expandable outer trampoline portion 832 is visible from the interior of the implant 800 in FIG. 88 and the inner circumferential valve portion 834 is visible in both FIGS. 90 and 92. As can be seen, the material 844 of the implant skirt 840 does not cover the wall-retaining petals 848, but it does project into the central orifices defined by each petal 848 in order to cover the proximal jack strut 812. The framework of the implant 800 is shown without the external coverings and valve material in FIGS. 89, 91, and 93 to expose the adjustable stent lattice 810, the skirt lattice 842, and the wall-retaining petals 848. Because the disconnect lumens 140 and the drive wires 150 connect from the side of the wall-retaining petals 848, the nosecone 120 is on the side of the implant skirt 840.

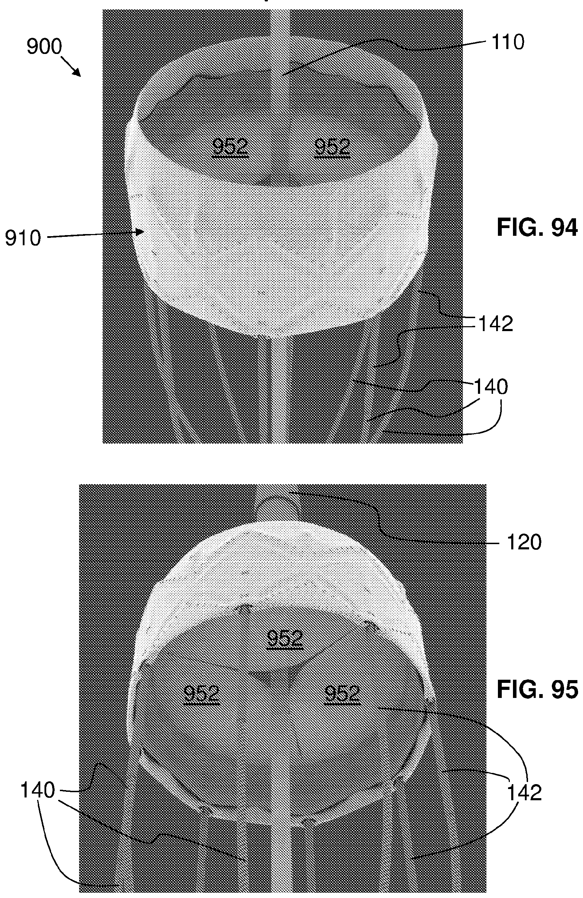

A fourth alternative configuration of a circular valve replacement implant 900 is shown in FIGS. 94 and 95. In contrast to the previous exemplary embodiments, the implant 900 has no exterior skirt, has no wall-retaining petals, and does not have the trampoline valve. The implant 900 has an overall circular shape and a tricuspid valve with leaflets 952 attached to the interior of the adjustable stent lattice 910.

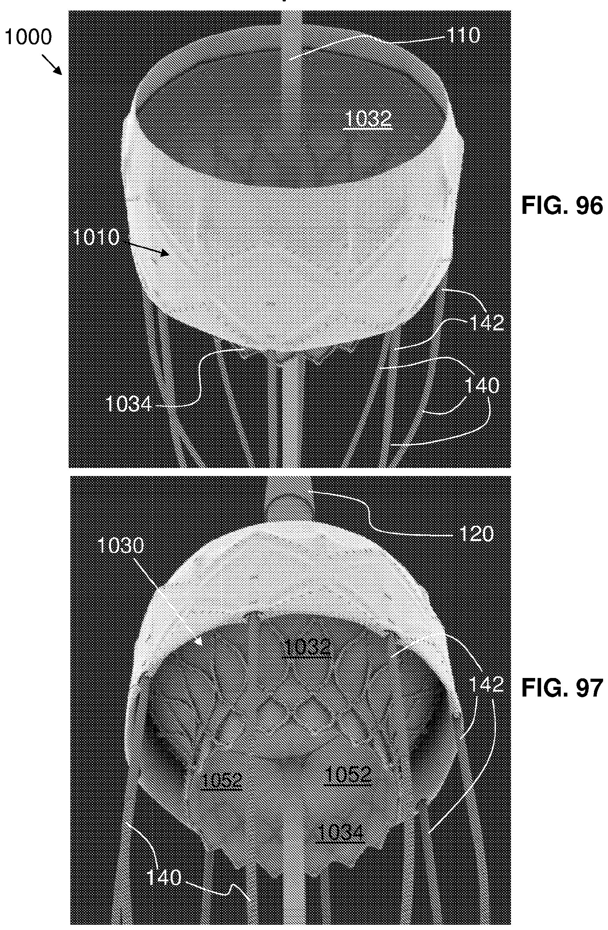

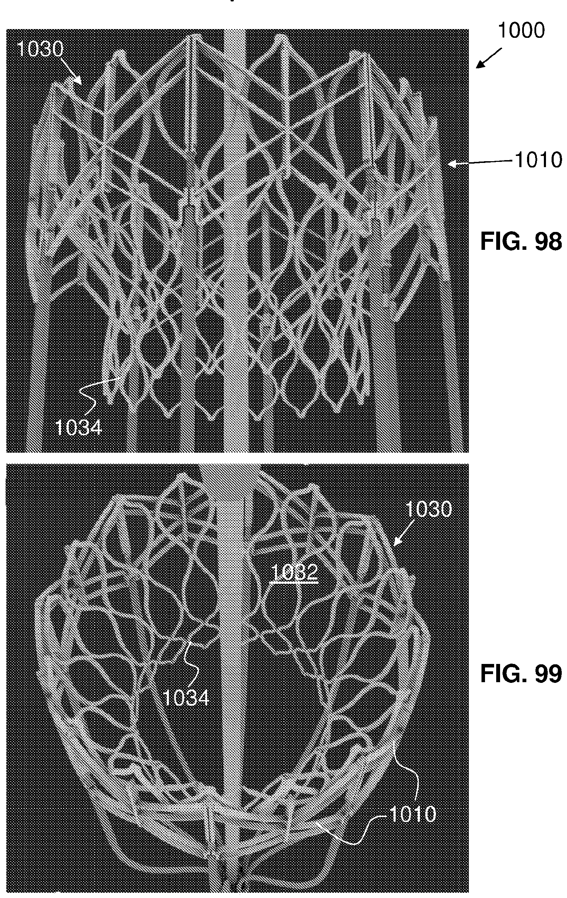

A fifth alternative configuration of a circular valve replacement implant 1000 is shown in FIGS. 96 to 99. In contrast to the previous exemplary embodiments, the implant 1000 has a self-expanding valve trampoline lattice 1030 but it does not have an exterior skirt or wall-retaining petals. The trampoline lattice 1030 has an inner circumferential valve portion 1034 on a side where the disconnect lumens 140 project out from the proximal end of the adjustable stent lattice 1010. The expandable outer trampoline portion 1032 is visible from the interior of the implant 1000 in FIG. 96 and the inner circumferential valve portion 1034 is visible in both FIGS. 96 and 97 with the internal leaflets 1052 is visible in FIG. 97. The framework of the implant 1000 is shown without the external coverings and valve material in FIGS. 98 and 99 to expose the adjustable stent lattice 1010 and the trampoline lattice 1030. Because the disconnect lumens 140 and the drive wires 150 connect from the side of the inner circumferential valve portion 1034, the nosecone 120 is on the side opposite the inner circumferential valve portion 1034.

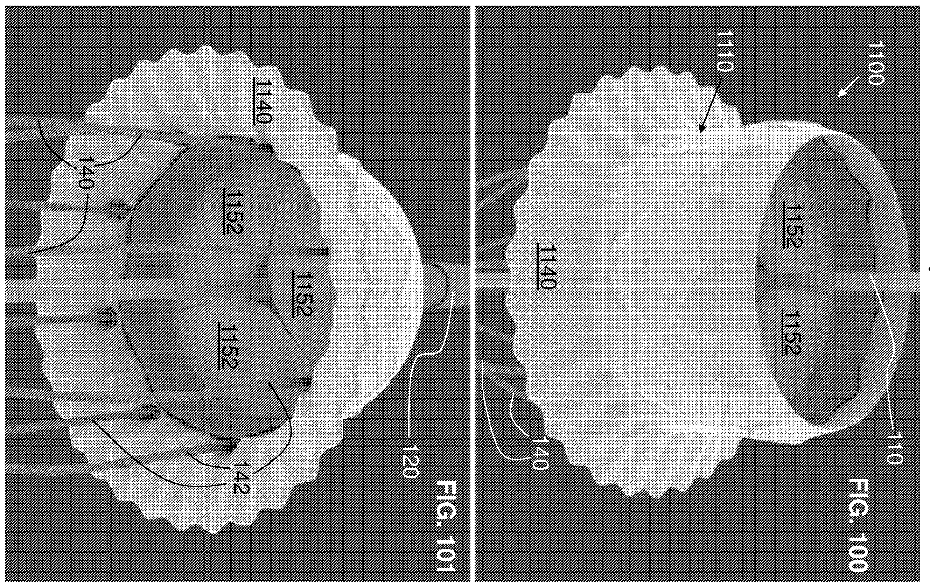

A sixth alternative configuration of a circular valve replacement implant 1100 is shown in FIGS. 100 to 102. In contrast to the previous exemplary embodiments, the implant 1100 has an exterior implant skirt 1140 but it does not have self-expanding valve trampoline lattice or wall-retaining petals. The valve contained within the adjustable stent lattice 1110 has three leaflets 1152, which are shown in FIGS. 100 and 101. The implant 1100 is installed from the side of the adjustable stent lattice 1110 having the implant skirt 1140, the side where the disconnect lumens 140 project out from the proximal end of the adjustable stent lattice 1110. Because the disconnect lumens 140 and the drive wires 150 connect from the side of the implant skirt 1140, the nosecone 120 is on the side opposite the implant skirt 1140, as shown in FIG. 101.

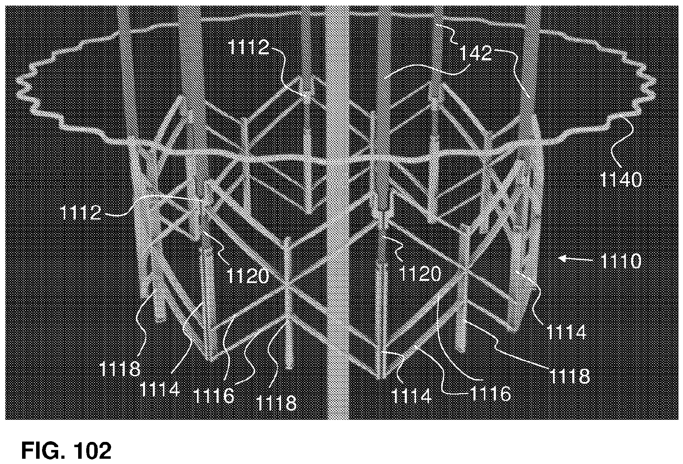

The framework of the implant 1100 is shown without the external coverings and valve material in FIG. 102 to expose the adjustable stent lattice 1110 and the lattice of the skirt 1140, as well as their various components. The implant skirt 1140, in this exemplary embodiment comprises only one part, an outer circumferential ring. This implant skirt 1140 does not connect to the adjustable stent lattice with intervening struts as in some of the above embodiments. The skirt 1140 is made of a material that is self-expanding and has a desired, pre-set shape (such as heat-set nitinol, for example). The adjustable stent lattice 1110 comprises sets of jack screw connectors, each set having a proximal jack strut 1112 and a distal jack strut 1114. Arms 1116 connect each of the proximal and distal jack struts 1112, 1114 to an intermediate strut 1118. Jack screws 1120 connect to the proximal and distal jack struts 1112, 1114 so that, when the jack screw 1120 is turned in one direction, the proximal and distal jack struts 1112, 114 separate from one another and, when the jack screw 1120 is turned in the other opposite direction, the proximal and distal jack struts 1112, 1114 move towards one another. This configuration can be achieved in various ways as described above and, therefore, this is not repeated here.

With such a configuration, rotation of the many jack screws 1120 in the strut-approaching direction causes the proximal and distal jack struts 1112, 1114 to move towards one another and, thereby, push the intermediate struts 1118 (which are disposed parallel to the jack screws 1120) away from the jack screw 1120 in a direction along the circumferential extent of the annulus of the adjustable stent lattice 1110. This relative movement of the intermediate strut 1118 and the jack screw assemblies causes expansion of the adjustable stent lattice 1110 when the proximal and distal jack struts 1112, 1114 move towards one another and causes contraction of the adjustable stent lattice 1110 when the proximal and distal jack struts 1112, 1114 move away from one another. Ideally, all of the jack screws 120 are rotated at the same speed to but such equal movement is not to be considered limiting.

In the exemplary embodiment of the adjustable stent lattice 1110 shown, there are eight pairs of jack struts 1112, 1114 and eight intermediate struts 1118. This number is merely exemplary and there can be, for example, only six of each or any other number desired including any number from 1 to 10. Connecting the pairs of jack struts 1112, 1114 and the intermediate struts 1118 are the laterally extending arms 1116, which, in this exemplary embodiment is two for each of the proximal and distal jack struts 1112, 1114, but this number is not limiting. As the adjustable stent lattice 1110 is either contracted or expanded, the arms 1116 each flex at their two endpoints, one at a respective intermediate strut 1118 and the other at a respective one of a pair of jack struts 1112, 1114. When the adjustable stent lattice 1110 is contracted (e.g., for installation into the delivery sheath 130), the arms 1116 move towards a longitudinal orientation (parallel to the jack screws and to the central axis of the lattice 1110. Conversely, when the adjustable stent lattice 1110 is expanded (e.g., for implantation), the arms 1116 angle away from the respective intermediate strut 1118 and one of the pair of jack struts 1112, 1114 in a circumferential orientation (perpendicular to the jack screws).

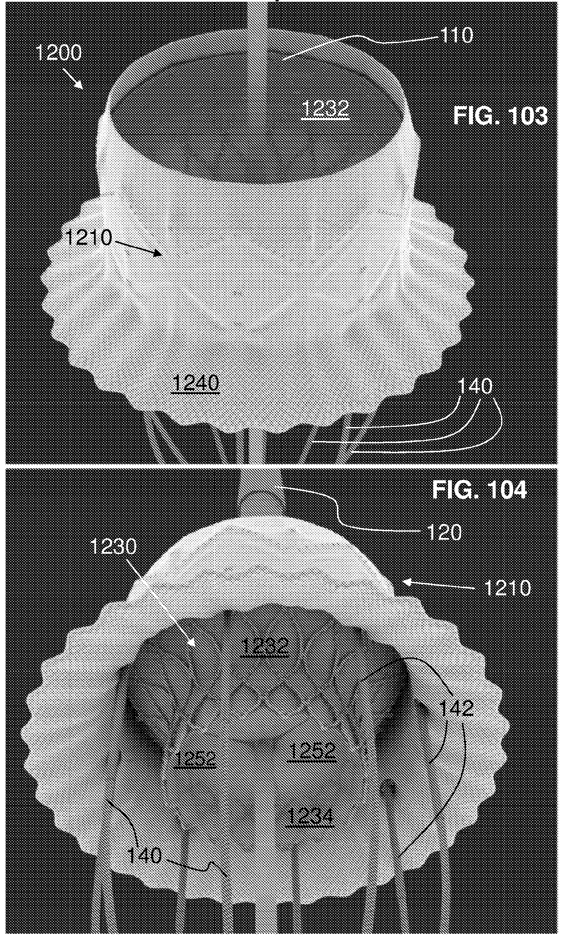

A seventh alternative configuration of a circular valve replacement implant 1200 is shown in FIGS. 103 and 104. In contrast to the previous exemplary embodiments, the implant 1200 has an exterior implant skirt 1240 and a self-expanding valve trampoline lattice 1230 but it does not have wall-retaining petals. The trampoline lattice 1230 has an inner circumferential valve portion 1234 on the side of the implant skirt 1240 where the disconnect lumens 140 project out from the proximal end of the adjustable stent lattice 1210. The expandable outer trampoline portion 1232 is visible from the interior of the implant 1200 in FIG. 103 and the inner circumferential valve portion 1234 is visible in FIG. 104 with the internal leaflets 1252 also visible there, three in number in this example. The implant 1200 is installed from the skirt side of the adjustable stent lattice 1210 where the inner circumferential valve portion 1234 exists and the side where the disconnect lumens 140 project out from the proximal end of the adjustable stent lattice 1210. Because the disconnect lumens 140 and the drive wires 150 connect from the side of the inner circumferential valve portion 1234, the nosecone 120 is on the side opposite the inner circumferential valve portion 1234.

In the above embodiments, memory shape and other metallic lattices were described. These lattices can have a material thickness of between 0.6 mm (.024") and 0.9 mm (.035") and any number therebetween, in particular between 0.7 mm (.028") and 0.8 mm (.032") and any number therebetween.

With mitral valve replacement implants having a trampoline valve, the number of sizes needed to cover the range of patient population is decreased from prior art TAVR replacement valves, which generally requires at least four sizes to be available for use. For the trampoline valves described herein, a 22 mm diameter valve can reside within a valve trampoline lattice having an expanded diameter starting at approximately 25 mm at its smallest to approximately 40 mm at its largest size. A 30 mm diameter valve can reside within a valve trampoline lattice having an expanded diameter starting at approximately 40 mm at its smallest to approximately 55 mm at its largest size. With a valve diameter range of between 25 mm and 55 mm, this means that the herein-described mitral valve implants having valve trampolines can cover the entire range of expected patient population with only two sizes.

The catheter sheaths required to implant these two are approximately 28 Fr to 32 Fr for the 25 mm to 40 mm size and 32 Fr to 35 Fr for the 40 mm to 55 mm size. Well within the desired range of catheters for such valve replacement procedures. It is further noted that if porcine pericardium is used for the valve leaflets, the size of the delivery sheath can be reduced, in particular, to 25 Fr to 29 Fr and 29 to 32 Fr, respectively.

It is noted that various individual features of the inventive processes and systems may be described only in one exemplary embodiment herein. The particular choice for description herein with regard to a single exemplary embodiment is not to be taken as a limitation that the particular feature is only applicable to the embodiment in which it is described. All features described herein are equally applicable to, additive, or interchangeable with any or all of the other exemplary embodiments described herein and in any combination or grouping or arrangement. In particular, use of a single reference numeral herein to illustrate, define, or describe a particular feature does not mean that the feature cannot be associated or equated to another feature in another drawing figure or description. Further, where two or more reference numerals are used in the figures or in the drawings, this should not be construed as being limited to only those embodiments or features, they are equally applicable to similar features or not a reference numeral is used or another reference numeral is omitted.

The foregoing description and accompanying drawings illustrate the principles, exemplary embodiments, and modes of operation of the systems and apparatuses. However, the systems, apparatuses, and methods should not be construed as being limited to the particular embodiments discussed above. Additional variations of the embodiments discussed above will be appreciated by those skilled in the art and the above-described embodiments should be regarded as illustrative rather than restrictive. Accordingly, it should be appreciated that variations to those embodiments can be made by those skilled in the art without departing from the scope of the systems and apparatuses as defined by the following claims.

## Claims

1. A mitral valve implant (200, 300, 400, 500), comprising:
a force-expanding mitral valve lattice (210) having an interior orifice;
a self-expanding valve trampoline lattice (230) attached at the interior orifice of the force-expanding mitral valve lattice (210);
**characterized in that** the force-expanding mitral valve lattice (210) comprises a plurality of jack screws (220) configured to adjust a configuration of the mitral valve lattice (210), wherein the force-expanding mitral valve lattice (210) is self-expandable to a first configuration and is force expandable from the first configuration to a second configuration.

2. The implant (200, 300, 400, 500) according to claim 1, wherein the force-expanding mitral valve lattice (210) is made of a shape memory material set shape to a given shape.

3. The implant (200, 300, 400, 500) according to claim 1, wherein the first configuration is circular or D-shaped and/or wherein the second configuration is circular or D-shaped.

4. The implant (200, 300, 400, 500) according to any one of the preceding claims, wherein the valve trampoline lattice (230) has a central region comprising valve leaflets (252), wherein the central region preferably is a cylindrical portion of the valve trampoline lattice (230) and the valve leaflets (252) are contained within the cylindrical portion.

5. The implant (200, 300, 400, 500) according to claim 4, wherein the valve trampoline lattice (230) comprises a D-shaped portion, wherein preferably the valve leaflets (252) have an inflow side and the D-shaped portion preferably is located on an inflow side of the valve leaflets (252).

6. The implant (200, 300, 400, 500) according to any one of the preceding claims, wherein the mitral valve lattice (210) has an exterior and further comprising an outwardly flaring implant skirt (240) attached to the exterior of the mitral valve lattice (210) and shaped to be positioned on a side of a native mitral valve (12).

7. The implant (200, 300, 400, 500) according to any one of the preceding claims, further comprising wall-retaining wires (248) attached to the mitral valve lattice (210) and shaped to be positioned on a side of the native mitral valve (12), wherein the wall-retaining wires (248) preferably are in the shape of petals (248) and have a pre-set, radially outward, memory shape to impart a force on the side of the native mitral valve (12) when the mitral valve lattice (210) is expanded within an annulus of the native mitral valve (12).

8. The implant (200, 300, 400, 500) according to claim 6 or 7, wherein:
the implant skirt (240) is a left ventricle or left atrium implant skirt shaped to be positioned on a ventricular side or atrial side of the native mitral valve (12), respectively, when the mitral valve lattice (210) is expanded within the annulus of the native mitral valve (12); and
the wall-retaining wires (248) respectively are left-atrium or left-ventricle wall-retaining wires shaped to be positioned on an atrial side or ventricular side of the native mitral valve (12), respectively, when the mitral valve lattice (210) is expanded within the annulus of the native mitral valve (12).

9. The implant (200, 300, 400, 500) according to any one of the preceding claims, wherein:
the mitral valve lattice (210) has an inlet end and an outlet end; and
the valve trampoline lattice (230) is attached to the inlet end or outlet end of the interior orifice.

10. A mitral valve implant system, comprising the implant (200, 300, 400, 500) of any one of the preceding claims and a delivery system (100) configured to actively control expansion and contraction of the implant (200, 300, 400, 500).

11. The system according to claim 10, wherein the delivery system (100) comprises a plurality of implant drive wires (150) and the implant drive wires (150) are temporarily connectable to jack screws (220) of the mitral valve lattice (210) such that rotation of the drive wires (150) in one direction forcibly expands the mitral valve lattice (210) and rotation of the drive wires (150) in a direction opposite the one direction forcibly contracts the mitral valve lattice (210).

12. A mitral heart valve implant system, comprising the implant (200, 300, 400, 500) of any one of claims 1 to 9 and
a valve delivery system (100) comprising:
a controller;
a guidewire lumen (110) connected to the controller and having a distal nosecone (120);
a hollow external sheath (130) surrounding the guidewire lumen (110), having a proximal end connected to the controller, and configured to retract proximally from an extended, valve-installed position;
a given number of implant drive wires (150) each having a distal drive wire connector (152); and
hollow connector lumens (140) equal in number to the given number and each:
respectively threaded on one of the drive wires (150); and
having a distal hollow connector sleeve;
the self-expanding and forcibly expanding mitral valve lattice (210) defining a central axis and comprising:
proximal and distal jack screw strut pairs (512, 514) equal in number to the given number and disposed parallel to the central axis;
intermediate struts (518) equal in number to the given number and disposed parallel to the central axis, each intermediate strut (518) disposed between two adjacent ones of the jack screw strut pairs (512, 514);
arms (516) respectively connecting adjacent ones of the jack screw strut pairs (512, 514) and the intermediate struts (518);
the plurality of jack screws (220):
each rotatably connected to one jack screw strut pair (512, 514); forming, together with the jack screw strut pairs (512, 514), the intermediate struts (518), and the arms (216), the adjustable stent lattice (210, 510) having a ventricle side and an atrial side;
configured to reversibly forcibly expand and contract the adjustable stent lattice (210, 510) between a compressed state and an enlarged state for implantation of the mitral valve frame into a native mitral valve (12); and
each having a driving connector (152) shaped to removably mate and connect to the distal drive wire connector (152) of one of the drive wires (150) and be held connected thereto when the hollow connector sleeve (140) is disposed about the mated driving connector (152) and distal drive wire connector such that rotation of the drive wires (150) correspondingly rotates the jack screws (220) to forcibly expand or contract the adjustable stent lattice (210, 510);
a self-expanding implant skirt (240) attached to the ventricle side of the adjustable stent lattice (210, 510), the implant skirt (240) configured to compress and be stored inside the external sheath (130) and, when released from the external sheath (130) at a native mitral valve (12), to self-expand and sealably position on tissue at a ventricular side of the native mitral valve (12);
wall-retaining wires (248) attached to the atrium side of the adjustable stent lattice (210, 510) and configured to compress and be stored inside the external sheath (130) and, when released from the external sheath (130) at a native mitral valve (12), to self-expand on tissue at an atrial side of the native mitral valve (12);
the self-expanding valve trampoline lattice (230) disposed inside and connected to the adjustable stent lattice (210, 510) and comprising:
an expandable outer trampoline portion (232) having a circumferential exterior connected to the interior of the adjustable stent lattice (210, 510) and a circumferential interior; and
an inner circumferential valve portion (234):
connected to the circumferential interior and extending inwardly from the circumferential interior to define an interior cylindrical portion; and
having a circular valve (250) with internal valve leaflets (252) disposed at the interior cylindrical portion.

13. The system according to claim 12, wherein:
the adjustable stent lattice (210, 510) has a pre-set D-shaped cross-section; and
the exterior of the expandable outer trampoline portion (232) is pre-set to a circumferential D-shape, wherein the D-shape preferably substantially approximates a D-shape of a native mitral valve (12) annulus.

14. A mitral heart valve implant system, comprising:
the implant (200, 300, 400, 500) of any one of claims 1 to 9,
the mitral valve lattice (210) having a pre-set D-shaped cross-sectional configuration, defining the internal orifice, and comprising a plurality of jack screws (220) configured to forcibly expand and contract the mitral valve lattice (210) reversibly between the first configuration corresponding to a compressed configuration and the second configuration corresponding to an enlarged configuration;
an outwardly flaring, self-expanding implant skirt (240) attached to an exterior of the mitral valve lattice (210), the implant skirt (240) shaped to be positioned on a ventricular side of a native mitral valve (12) to secure the mitral valve lattice (210) in the annulus of the native mitral valve (12);
radially outwardly biased wall-retaining wires (248) attached to the mitral valve lattice (210) and shaped to be positioned on an atrial side of the native mitral valve (12) to secure the mitral valve lattice (210) in the annulus of the native mitral valve (12);
the self-expanding valve trampoline lattice (230) containing interior valve leaflets (252), the valve trampoline lattice (230) having a D-shape portion attached to the mitral valve lattice (210) and a substantially cylindrical interior portion, wherein the valve leaflets (252) are attached to the substantially cylindrical interior portion; and
a delivery system (100) comprising a plurality of implant drive wires (150) temporarily connectable to the jack screws (220) such that, when connected, rotation of the drive wires (150) in one direction forcibly expands the mitral valve lattice (210) towards the enlarged configuration and rotation of the drive wires (150) in a direction opposite the one direction forcibly contracts the mitral valve lattice (210) towards the compressed configuration.

## Patentansprüche

1. Mitralklappenimplantat (200, 300, 400, 500), aufweisend:
ein kraftexpandierendes Mitralklappengitter (210) mit einer inneren Öffnung;
ein selbstexpandierendes Herzklappen-Trampolingitter (230), das an der inneren Öffnung des kraftexpandierenden Mitralklappengitters (210) angebracht ist;
**dadurch gekennzeichnet, dass** das kraftexpandierende Mitralklappengitter (210) eine Mehrzahl von Stellschrauben (220) umfasst, die zum Einstellen einer Konfiguration des Mitralklappengitters (210) ausgebildet sind, wobei das kraftexpandierende Mitralklappengitter (210) zu einer ersten Konfiguration selbstexpandierbar und von der ersten Konfiguration zu einer zweiten Konfiguration kraftexpandierbar ist.

2. Implantat (200, 300, 400, 500) nach Anspruch 1, wobei das kraftexpandierende Mitralklappengitter (210) aus einem Formgedächtnismaterial gebildet ist, das auf eine bestimmte Form eingestellt ist.

3. Implantat (200, 300, 400, 500) nach Anspruch 1, wobei die erste Konfiguration kreisförmig oder D-förmig ist und/oder wobei die zweite Konfiguration kreisförmig oder D-förmig ist.

4. Implantat (200, 300, 400, 500) nach einem der vorstehenden Ansprüche, wobei das Herzklappen-Trampolingitter (230) einen zentralen Bereich hat, der Klappensegel (252) aufweist, wobei der zentrale Bereich vorzugsweise ein zylindrischer Abschnitt des Herzklappen-Trampolingitters (230) ist und die Klappensegel (252) innerhalb des zylindrischen Abschnitts aufgenommen sind.

5. Implantat (200, 300, 400, 500) nach Anspruch 4, wobei das Herzklappen-Trampolingitter (230) einen D-förmigen Abschnitt aufweist, wobei die Klappensegel (252) vorzugsweise eine Einströmseite aufweisen und der D-förmige Abschnitt vorzugsweise auf einer Einströmseite der Klappensegel (252) angeordnet ist.

6. Implantat (200, 300, 400, 500) nach einem der vorstehenden Ansprüche, wobei das Mitralklappengitter (210) eine Außenseite aufweist und ferner aufweisend eine sich nach außen erweiternde Implantatschürze (240), die an der Außenseite des Mitralklappengitters (210) angebracht und geformt ist, um an einer Seite einer nativen Mitralklappe (12) positioniert zu werden.

7. Implantat (200, 300, 400, 500) nach einem der vorstehenden Ansprüche, ferner aufweisend Wandhaltedrähte (248), die an dem Mitralklappengitter (210) angebracht und geformt sind, um an einer Seite der nativen Mitralklappe (12) positioniert zu werden, wobei die Wandhaltedrähte (248) vorzugsweise die Form von Blütenblättern (248) und eine voreingestellte, radial nach außen gerichtete Gedächtnisform haben, um eine Kraft auf die Seite der nativen Mitralklappe (12) auszuüben, wenn das Mitralklappengitter (210) in einem Annulus der nativen Mitralklappe (12) expandiert wird.

8. Implantat (200, 300, 400, 500) nach Anspruch 6 oder 7, wobei:
die Implantatschürze (240) eine Implantatschürze für das linke Ventrikel oder das linke Atrium ist, die geformt ist, um auf der ventrikulären Seite beziehungsweise der atrialen Seite der nativen Mitralklappe (12) positioniert zu werden, wenn das Mitralklappengitter (210) innerhalb des Annulus der nativen Mitralklappe (12) expandiert wird; und
die Wandhaltedrähte (248) entsprechende linksatrium- oder linksventrikelseitige Wandhaltedrähte sind, die geformt sind, um auf einer atrialen Seite beziehungsweise ventrikulären Seite der nativen Mitralklappe (12) positioniert zu werden, wenn das Mitralklappengitter (210) innerhalb des Annulus der nativen Mitralklappe (12) expandiert wird.

9. Implantat (200, 300, 400, 500) nach einem der vorstehenden Ansprüche, wobei:
das Mitralklappengitter (210) ein Einlassende und ein Auslassende hat; und
wobei das Herzklappen-Trampolingitter(230) am Einlassende oder Auslassende der inneren Öffnung angebracht ist.

10. Mitralklappen-Implantatsystem, aufweisend das Implantat (200, 300, 400, 500) nach einem der vorstehenden Ansprüche und ein Zuführsystem (100), das zum aktiven Steuern der Expansion und Kontraktion des Implantats (200, 300, 400, 500) ausgebildet ist.

11. System nach Anspruch 10, wobei das Zuführsystem (100) eine Mehrzahl von Implantatantriebsdrähten (150) aufweist und die Implantatantriebsdrähte (150) tempo-rär mit Stellschrauben (220) des Mitralklappengitters (210) verbindbar sind, so dass eine Rotation der Antriebsdrähte (150) in eine Richtung das Mitralklappengitter (210) kraftgetrieben expandiert und eine Rotation der Antriebsdrähte (150) in eine der einen Richtung entgegengesetzte Richtung das Mitralklappengitter (210) kraftgetrieben kontrahiert.

12. Mitralklappen-Implantatsystem, aufweisend das Implantat (200, 300, 400, 500) nach einem der Ansprüche 1 bis 9 und ein Herzklappenzuführsystem (100), aufweisend:
eine Steuerung;
ein Führungsdrahtlumen (110), das mit der Steuerung verbunden ist und einen distalen Nasenkonus (120) hat;
eine hohle externe Hülle (130), die das Führungsdrahtlumen (110) umgibt, ein mit der Steuerung verbundenes proximales Ende hat und dazu ausgebildet ist, aus einer ausgefahrenen, herzklappeninstallierten Position proximal zurückzuziehen;
eine bestimmte Zahl von Implantatantriebsdrähten (150), die jeweils einen distalen Antriebsdrahtverbinder (152) haben; und
hohle Verbindungslumen (140), in gleicher Anzahl zu der bestimmten Zahl und jeweils:
auf einen entsprechenden der Antriebsdrähte (150) geschraubt;
und mit einer distalen hohlen Verbindungshülse;
wobei das selbstexpandierende und kraftexpandierende Mitralklappengitter (210) eine Zentralachse definiert und aufweist:
proximale und distale Stellschraubenstrebenpaare (512, 514) in gleicher Anzahl zur bestimmten Zahl und parallel zur Zentralachse angeordnet;
Zwischenstreben (518) in gleicher Anzahl zur bestimmten Zahl und parallel zur Zentralachse angeordnet, wobei jede Zwischenstrebe (518) zwischen zwei benachbarten der Stellschraubenstrebenpaare (512, 514) angeordnet ist;
Arme (516), die jeweils benachbarte der Stellschraubenstrebenpaare (512, 514) und der Zwischenstreben (518) verbinden;
die Mehrzahl von Stellschrauben (220):
wobei jede drehbar mit einem Stellschraubenstrebenpaar (512, 514) verbunden ist;
wobei diese zusammen mit den Stellschraubenstrebenpaaren (512, 514), den Zwischenstreben (518) und den Armen (216) das einstellbare Stentgitter (210, 510) bilden, wobei das einstellbare Stentgitter (210, 510) eine ventrikuläre Seite und eine atriale Seite aufweist;
wobei diese dazu ausgebildet sind, das einstellbare Stentgitter (210, 510) reversibel kraftgetrieben zwischen einem komprimierten Zustand und einem vergrößerten Zustand zur Implantation des Mitralklappenrahmens in eine native Mitralklappe (12) zu expandieren und zu kontrahieren;
und wobei diese jeweils einen Antriebsverbinder (152) haben, der geformt ist, um reversibel mit dem distalen Antriebsdrahtverbinder (152) eines der Antriebsdrähte (150) zu koppeln und zu verbinden und mit diesem verbunden zu bleiben, wenn die hohle Verbindungshülse (140) um den gekoppelten Antriebsverbinder (152) und distalen Antriebsdrahtverbinderangeordnet ist, so dass eine Rotation der Antriebsdrähte (150) entsprechend die Stellschrauben (220) rotiert, um das einstellbare Stentgitter (210, 510) kraftgetrieben zu expandieren oder zu kontrahieren;
eine selbstexpandierende Implantatschürze (240), die an der ventrikulären Seite des einstellbaren Stentgitters (210, 510) angebracht ist, wobei die Implantatschürze (240) dazu ausgebildet ist, komprimiert und innerhalb der externen Hülle (130) gelagert zu werden, und sich selbst zu expandieren und dichtend an Gewebe an einer ventrikulären Seite der nativen Mitralklappe zu positionieren (12), wenn sie aus der externen Hülle (130) freigesetzt wird;
Wandhaltedrähte (248), die an der atrialen Seite des einstellbaren Stentgitters (210, 510) befestigt und dazu ausgebildet sind, komprimiert und innerhalb der externen Hülle (130) gelagert zu werden, und sich selbst auf Gewebe an einer atrialen Seite der nativen Mitralklappe (12) zu expandieren, wenn sie an einer nativen Mitralklappe (12) aus der externen Hülle (130) freigesetzt werden;
das selbstexpandierende Herzklappen-Trampolingitter (230), das im Inneren des einstellbaren Stentgitters (210, 510) angeordnet und damit verbunden ist, und aufweisend:
einen expandierbaren äußeren Trampolinabschnitt (232) mit einem umlaufenden Äußeren, das mit dem Inneren des einstellbaren Stentgitters (210, 510) verbunden ist, und einem umlaufenden Inneren; und einem inneren umlaufenden Ventilabschnitt (234):
der mit dem umlaufenden Inneren verbunden ist und sich von dem umlaufenden Inneren nach innen erstreckt, um einen inneren zylindrischen Abschnitt zu definieren; und
der ein kreisförmiges Ventil (250) mit inneren Klappensegeln (252) hat, die an dem inneren zylindrischen Abschnitt angeordnet sind.

13. System nach Anspruch 12, wobei:
das einstellbare Stentgitter (210, 510) einen voreingestellten D-förmigen Querschnitt aufweist; und
das Äußere des expandierbaren äußeren Trampolinabschnitts (232) auf eine umlaufende D-Form voreingestellt ist, wobei die D-Form vorzugsweise im Wesentlichen eine D-Form eines nativen Mitralannulus (12) annähert.

14. Mitralklappen-Implantatsystem, aufweisend:
das Implantat (200, 300, 400, 500) nach einem der Ansprüche 1 bis 9,
wobei das Mitralklappengitter (210) eine voreingestellte D-förmige Querschnittskonfiguration hat, die die innere Öffnung definiert, und eine Mehrzahl von Stellschrauben (220) umfasst, die dazu ausgebildet sind, das Mitralklappengitter (210) reversibel zwischen der ersten Konfiguration, die einer komprimierten Konfiguration entspricht, und der zweiten Konfiguration, die einer aufgeweiteten Konfiguration entspricht, kraftgetrieben zu expandieren und zu kontrahieren;
eine sich nach außen erweiternde, selbstexpandierende Implantatschürze (240), die an einem Äußeren des Mitralklappengitters (210) angebracht ist, wobei die Implantatschürze (240) geformt ist, um an einer ventrikulären Seite einer nativen Mitralklappe (12) positioniert zu werden, um das Mitralklappengitter (210) im Annulus der nativen Mitralklappe (12) zu sichern;
radial nach außen vorgespannte Wandhaltedrähte (248), die an dem Mitralklappengitter (210) angebracht und geformt sind, um an einer atrialen Seite der nativen Mitralklappe (12) positioniert zu werden, um das Mitralklappengitter (210) in dem Annulus der nativen Mitralklappe (12) zu sichern;
das selbstexpandierende Herzklappen-Trampolingitter(230), das innere Klappensegel (252) enthält, wobei das Herzklappen-Trampolingitter (230) einen D-förmigen Abschnitt, der an dem Mitralklappengitter (210) angebracht ist, und einen im Wesentlichen zylindrischen inneren Abschnitt aufweist, wobei die Klappensegel (252) an dem im Wesentlichen zylindrischen inneren Abschnitt angebracht sind; und
ein Zuführsystem (100), das eine Mehrzahl von Implantatantriebsdrähten (150) aufweist, die temporär mit den Stellschrauben (220) verbindbar sind, so dass, wenn sie verbunden sind, eine Rotation der Antriebsdrähte (150) in eine Richtung das Mitralklappengitter (210) kraftgetrieben in Richtung der aufgeweiteten Konfiguration expandiert und eine Rotation der Antriebsdrähte (150) in einer der einen Richtung entgegengesetzten Richtung das Mitralklappengitter (210) kraftgetrieben in Richtung der komprimierten Konfiguration kontrahiert.

## Revendications

1. Implant (200, 300, 400, 500) de valvule mitrale, comprenant :
un treillis (210) de valvule mitrale déployable sous l'effet d'une force présentant un orifice intérieur ;
un treillis trampoline (230) de valvule autodéployable fixé au niveau de l'orifice intérieur du treillis (210) de valvule mitrale déployable sous l'effet d'une force ;
**caractérisé en ce que** le treillis (210) de valvule mitrale déployable sous l'effet d'une force comprend une pluralité de vérins à vis (220) conçus pour ajuster une configuration du treillis (210) de valvule mitrale, le treillis (210) de valvule mitrale déployable sous l'effet d'une force étant autodéployable vers une première configuration et étant déployable sous l'effet d'une force à partir de la première configuration vers une seconde configuration.

2. Implant (200, 300, 400, 500) selon la revendication 1, le treillis (210) de valvule mitrale déployable sous l'effet d'une force étant fabriqué en un matériau à mémoire de forme qui lui confère une forme donnée.

3. Implant (200, 300, 400, 500) selon la revendication 1, la première configuration étant circulaire ou en forme de D et/ou la seconde configuration étant circulaire ou en forme de D.

4. Implant (200, 300, 400, 500) selon l'une quelconque des revendications précédentes, le treillis trampoline (230) de valvule présentant une région centrale comprenant des feuillets valvulaires (252), la région centrale étant de préférence une partie cylindrique du treillis trampoline (230) de valvule et les feuillets valvulaires (252) étant contenus à l'intérieur de la partie cylindrique.

5. Implant (200, 300, 400, 500) selon la revendication 4, le treillis trampoline (230) de valvule comprenant une partie en forme de D, les feuillets valvulaires (252) présentant de préférence un côté entrée et la partie en forme de D étant de préférence située sur un côté entrée des feuillets valvulaires (252).

6. Implant (200, 300, 400, 500) selon l'une quelconque des revendications précédentes, le treillis (210) de valvule mitrale présentant un extérieur et comprenant en outre une collerette (240) d'implant s'évasant vers l'extérieur fixée à l'extérieur du treillis (210) de valvule mitrale et formée pour être positionnée sur un côté d'une valvule mitrale native (12).

7. Implant (200, 300, 400, 500) selon l'une quelconque des revendications précédentes, comprenant en outre des fils de retenue (248) de paroi fixés au treillis (210) de valvule mitrale et formés pour être positionnés sur un côté de la valvule mitrale native (12), les fils de retenue (248) de paroi se présentant de préférence sous la forme de pétales (248) et présentant une forme de mémoire prédéfinie radialement vers l'extérieur destinée à transmettre une force sur le côté de la valvule mitrale native (12) lorsque le treillis (210) de valvule mitrale est déployé à l'intérieur d'un anneau de la valvule mitrale native (12).

8. Implant (200, 300, 400, 500) selon la revendication 6 ou 7 :
la collerette (240) d'implant étant une collerette d'implant de ventricule gauche ou d'oreillette gauche formée pour être positionnée respectivement sur un côté ventriculaire ou un côté auriculaire de la valvule mitrale native (12) lorsque le treillis (210) de valvule mitrale est déployé à l'intérieur de l'anneau de la valvule mitrale native (12) ; et
les fils de retenue (248) de paroi étant respectivement des fils de retenue de paroi d'oreillette gauche ou de ventricule gauche formés pour être positionnés respectivement sur un côté auriculaire ou un côté ventriculaire de la valvule mitrale native (12) lorsque le treillis (210) de valvule mitrale est déployé à l'intérieur de l'anneau de la valvule mitrale native (12).

9. Implant (200, 300, 400, 500) selon l'une quelconque des revendications précédentes :
le treillis (210) de valvule mitrale présentant une extrémité d'entrée et une extrémité de sortie ; et
le treillis trampoline (230) de valvule étant fixé à l'extrémité d'entrée ou à l'extrémité de sortie de l'orifice intérieur.

10. Système d'implant de valvule mitrale, comprenant l'implant (200, 300, 400, 500) selon l'une quelconque des revendications précédentes et un système de pose (100) conçu pour commander activement le déploiement et la contraction de l'implant (200, 300, 400, 500).

11. Système selon la revendication 10, le système de pose (100) comprenant une pluralité de fils d'entraînement (150) d'implant et les fils d'entraînement (150) d'implant pouvant être reliés temporairement à des vérins à vis (220) du treillis (210) de valvule mitrale de telle sorte que la rotation des fils d'entraînement (150) dans un sens déploie à force le treillis (210) de valvule mitrale et la rotation des fils d'entraînement (150) dans un sens opposé audit un sens contracte à force le treillis (210) de valvule mitrale.

12. Système d'implant de valvule cardiaque mitrale, comprenant l'implant (200, 300, 400, 500) selon l'une quelconque des revendications 1 à 9 et
un système de pose (100) de valvule comprenant :
un dispositif de commande ;
une lumière (110) à fil-guide reliée au dispositif de commande et présentant une pointe avant distale (120) ;
une gaine externe creuse (130) entourant la lumière (110) à fil-guide, présentant une extrémité proximale reliée au dispositif de commande et conçue pour se rétracter de manière proximale à partir d'une position étendue de valvule installée ;
un nombre donné de fils d'entraînement (150) d'implant présentant chacun un raccord distal (152) de fil d'entraînement ; et
des lumières creuses (140) de raccord égales en nombre au nombre donné et chacune :
étant enfilée respectivement sur l'un des fils d'entraînement (150) ; et
présentant un manchon creux de raccord distal ;
le treillis (210) de valvule mitrale autodéployable et se déployant à force définissant un axe central et comprenant :
des paires d'entretoises (512, 514) proximales et distales à vérins à vis égales en nombre au nombre donné et disposées parallèlement à l'axe central ;
des entretoises intermédiaires (518) égales en nombre au nombre donné et disposées parallèlement à l'axe central, chaque entretoise intermédiaire (518) étant disposée entre deux paires adjacentes des paires d'entretoises (512, 514) à vérins à vis ;
des bras (516) reliant respectivement des paires adjacentes des paires d'entretoises (512, 514) à vérins à vis et les entretoises intermédiaires (518) ;
la pluralité de vérins à vis (220) :
étant reliés chacun de manière rotative à une paires d'entretoises (512, 514) à vérins à vis ;
formant, conjointement avec les paires d'entretoises (512, 514) à vérins à vis, les entretoises intermédiaires (518) et les bras (216), le treillis (210, 510) d'endoprothèse ajustable présentant un côté ventriculaire et un côté auriculaire ;
étant conçus pour déployer et contracter à force de manière réversible le treillis (210, 510) d'endoprothèse ajustable entre un état comprimé et un état agrandi en vue de l'implantation du cadre de valvule mitrale dans une valvule mitrale native (12) ; et
présentant chacun un raccord d'entraînement (152) formé pour s'accoupler et se raccorder de manière amovible au raccord distal (152) de fil d'entraînement de l'un des fils d'entraînement (150) et pour être maintenu raccordé à celui-ci lorsque le manchon creux (140) de raccord est disposé autour du raccord d'entraînement (152) accouplé et du raccord distal de fil d'entraînement de telle sorte que la rotation des fils d'entraînement (150) fait tourner de manière correspondante les vérins à vis (220) afin de déployer ou de contracter à force le treillis (210, 510) d'endoprothèse ajustable ;
une collerette (240) d'implant autodéployable fixée au côté ventriculaire du treillis (210, 510) d'endoprothèse ajustable, la collerette (240) d'implant étant conçue pour se comprimer et être stockée à l'intérieur de la gaine externe (130) et, lorsqu'elle est libérée de la gaine externe (130) au niveau d'une valvule mitrale native (12), pour s'autodéployer et se positionner de manière étanche sur un tissu au niveau d'un côté ventriculaire de la valvule mitrale native (12) ;
des fils de retenue (248) de paroi fixés au côté auriculaire du treillis (210, 510) d'endoprothèse ajustable et conçus pour se comprimer et être stockés à l'intérieur de la gaine externe (130) et, lorsqu'ils sont libérés de la gaine externe (130) au niveau d'une valvule mitrale native (12), pour s'autodéployer sur un tissu au niveau d'un côté auriculaire de la valvule mitrale native (12) ;
le treillis trampoline (230) de valvule autodéployable disposé à l'intérieur et relié au treillis (210, 510) d'endoprothèse ajustable et comprenant :
une partie (232) de trampoline externe déployable présentant un extérieur circonférentiel relié à l'intérieur du treillis (210, 510) d'endoprothèse ajustable et un intérieur circonférentiel ; et
une partie (234) de valvule circonférentielle interne :
reliée à l'intérieur circonférentiel et s'étendant vers l'intérieur à partir de l'intérieur circonférentiel afin de définir une partie cylindrique intérieure ; et
présentant une valvule circulaire (250) avec des feuillets valvulaires (252) internes disposés au niveau de la partie cylindrique intérieure.

13. Système selon la revendication 12 :
le treillis (210, 510) d'endoprothèse ajustable présentant une section transversale en forme de D prédéfinie ; et
l'extérieur de la partie (232) de trampoline externe déployable étant prédéfini selon une forme circonférentielle de D, la forme de D s'approchant de préférence sensiblement d'une forme de D d'un anneau de valvule mitrale native (12).

14. Système d'implant de valvule cardiaque mitrale, comprenant :
l'implant (200, 300, 400, 500) selon l'une quelconque des revendications 1 à 9,
le treillis (210) de valvule mitrale présentant une configuration de section transversale en forme de D prédéfinie, définissant l'orifice interne et comprenant une pluralité de vérins à vis (220) conçus pour déployer et contracter à force le treillis (210) de valvule mitrale de manière réversible entre la première configuration correspondant à une configuration comprimée et la seconde configuration correspondant à une configuration agrandie ;
une collerette (240) d'implant autodéployable s'évasant vers l'extérieur fixée à un extérieur du treillis (210) de valvule mitrale, la collerette (240) d'implant étant formée pour être positionnée sur un côté ventriculaire d'une valvule mitrale native (12) afin de fixer le treillis (210) de valvule mitrale dans l'anneau de la valvule mitrale native (12) ;
des fils de retenue (248) de paroi sollicités radialement vers l'extérieur fixés au treillis (210) de valvule mitrale et formés pour être positionnés sur un côté auriculaire de la valvule mitrale native (12) afin de fixer le treillis (210) de valvule mitrale dans l'anneau de la valvule mitrale native (12) ;
le treillis trampoline (230) de valvule autodéployable contenant des feuillets valvulaires (252) intérieurs, le treillis trampoline (230) de valvule présentant une partie en forme de D fixée au treillis (210) de valvule mitrale et une partie intérieure sensiblement cylindrique, les feuillets valvulaires (252) étant fixés à la partie intérieure sensiblement cylindrique ; et
un système de pose (100) comprenant une pluralité de fils d'entraînement (150) d'implant pouvant être reliés temporairement aux vérins à vis (220) de telle sorte que, lorsqu'ils sont reliés, la rotation des fils d'entraînement (150) dans un sens déploie à force le treillis (210) de valvule mitrale vers la configuration agrandie et la rotation des fils d'entraînement (150) dans un sens opposé audit un sens contracte à force le treillis (210) de valvule mitrale vers la configuration comprimée.
